(19) Europäisches Patentamt
European Patent Office
Office européen des brevets

(11) Veröffentlichungsnummer: **0 430 004 A2**

# (12) EUROPÄISCHE PATENTANMELDUNG

(21) Anmeldenummer: **90122030.1**

(51) Int. Cl.[5]: **C07D 221/14**, A01N 25/32

(22) Anmeldetag: **17.11.90**

(30) Priorität: **29.11.89 DE 3939379**
**07.07.90 DE 4021654**

(43) Veröffentlichungstag der Anmeldung:
**05.06.91 Patentblatt 91/23**

(84) Benannte Vertragsstaaten:
**AT CH DE ES FR GB IT LI NL SE Patentblatt**

(71) Anmelder: **BASF Aktiengesellschaft**
**Carl-Bosch-Strasse 38**
**W-6700 Ludwigshafen(DE)**

(72) Erfinder: **Saupe, Thomas, Dr.**
**Kressenwiesenweg 13**
**W-6902 Sandhausen(DE)**
Erfinder: **Meyer, Norbert, Dr.**
**Dossenheimer Weg 22**
**W-6802 Ladenburg(DE)**
Erfinder: **Plath, Peter, Dr.**
**Hans-Balcke-Strasse 13**
**W-6710 Frankenthal(DE)**
Erfinder: **Schirmer, Ulrich, Dr.**
**Berghalde 79**
**W-6900 Heidelberg(DE)**
Erfinder: **Wuerzer, Bruno, Dr.**
**Ruedigerstrasse 13**
**W-6701 Otterstadt(DE)**
Erfinder: **Westphalen, Karl-Otto, Dr.**
**Mausbergweg 58**
**W-6720 Speyer(DE)**
Erfinder: **Patsch, Manfred, Dr.**
**Fritz-Wendel-Strasse 4**
**W-6706 Wachenheim(DE)**
Erfinder: **Pfister, Juergen, Dr.**
**Ziegelofenweg 1**
**W-6720 Speyer(DE)**

## (54) 1,8-Naphthalindicarbonsäureimide als Antidots.

(57) 1,8-Naphthalindicarbonsäureimide Ia

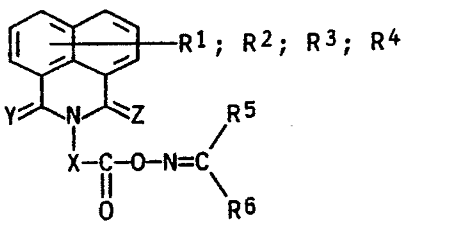

Ia

($R^1$-$R^4$ = H, Halogen, OH, SH, CN, SCN, $NO_2$, geg. halogeniertes Alkyl, Alkenyl, Alkinyl, Cycloalkyl, Cycloalkoxy, $NH_2$, Alkylamino, Dialkylamino, Aminoalkyl, Alkylaminoalkyl, Dialkylaminoalkyl, Alkoxy, Alkylthio, Alkoxyalkyl, Alkylthioalkyl, Alkylcarbonyl, Alkylcarbonyloxy, Alkoxycarbonyl, COOH, Hydroxycarbonylalkyl, Hydroxycarbonylalkoxy, Hydroxycarbonylalkylthio, $SO_3H$, Alkylsulfinyl, Alkylsulfonyl, Alkoxysulfonyl, $SO_2NH_2$, Alkylaminosulfonyl, Dialkylaminosulfonyl, geg. subst. $NHNH_2$, $C_6H_5$, Phenylalkyl, Naphthyl, Naphthylalkyl, Heteroaryl, Heteroarylalkyl, Phenoxy, Naphthoxy, Phenylthio, Naphthylthio, Phenoxycarbonyl, Naphthoxycarbonyl, Phenylcarbonyl, Naphthylcarbonyl, Phenylcarbonyloxy, Naphthylcarbonyloxy, Phenylalkoxy, Naphthylalkoxy, Heteroarylalkoxy, Phenylsulfinyl, Naphthylsulfinyl, Heteroarylsulfinyl, Phenylsulfonyl, Naphthylsulfonyl, Heteroarylsulfonyl, Phenoxysulfonyl, Naphthoxysulfonyl, Heteroaryloxysulfonyl, wobei alle Arylteile weitere Substituenten tragen können; x = geg. subst. Alkylenkette; $R^5$ = H, CN, Alkyl, Alkenyl, Alkoxyalkyl, Alkylthioalkyl, Alkoxycarbonyl, Acetyl,

EP 0 430 004 A2

Cycloalkyl, Benzoyl, Naphthoyl, Heteroaroyl, $C_6H_5$, Naphthyl, Phenylalkyl, Naphthylalkyl, Heteroaryl, Heteroarylalkyl, wobei die Arylteile der letztgenannten 6 Reste weitere Subst. tragen können; $R^6$ = H, CN, geg. halogeniertes Alkyl, Alkoxy, Alkoxyalkyl, Diazolylmethyl, Triazolylmethyl, Alkoxycarbonyl, Furyl, Tetrahydrofuryl, Thienyl, Tetrahydropyranyl, Tetrahydrothiopyranyl, Dihydropyranyl, Dihydrothiopyranyl; $R^6$ = $C_6H_5$, wenn $R^5$ = H, $CH_3$, $CH_3CO$; $R^5$ + $R^6$ + das gemeinsame C-Atom = Cycloalkyl, Oxocyclohexadienyl, ein geg. subst. 5-/6-gliedriger Ring, der ungesättigt sein kann und O oder S als Heteroatome enthalten kann; Y,Z = O, S; außer $R^5$ und $R^6$ gleichzeitig H) sowie im Falle $R^1$ - $R^4$ = saure Gruppen die pflanzenverträglichen Salze von Ia und herbizide Mittel, die 2-(4-Heteroaryloxy)- oder 2-(4-Aryloxy)-phenoxyessigsäure-oder -propionsäurederivate und/oder Cyclohexenonderivate als herbizide Wirkstoffe und 1,8-Naphthalindicarbonsäureimide Ia und/oder Ib

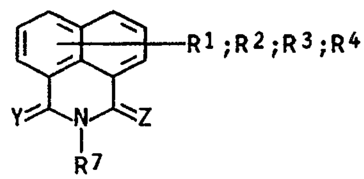

Ib

($R^7$ = verschiedene organische Reste),
und/oder Ic

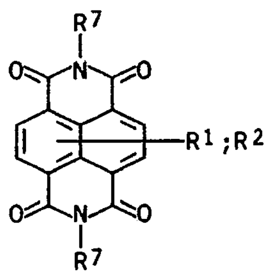

Ic

sowie im Falle saurer Endgruppen die pflanzenverträglichen Salze von Ia und/oder Ib und/oder Ic, als Antidots enthalten.

## 1,8-NAPHTHALINDICARBONSÄUREIMIDE ALS ANTIDOTS

Die vorliegende Erfindung betrifft neue substituierte 1,8-Naphthalindicarbonsäureimide der allgemeinen Formel Ia

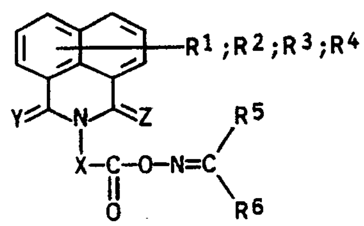

Ia

in der die Substituenten die folgende Bedeutung haben:

$R^1$ - $R^4$ Wasserstoff, Halogen, Hydroxy, Mercapto, Cyano, Thiocyanato, Nitro, $C_1$-$C_6$-Alkylgruppen, die unsubstituiert oder partiell oder vollständig halogeniert sein können, $C_2$-$C_6$-Alkenylgruppen, $C_2$-$C_6$-Alkinylgruppen, $C_3$-$C_8$-Cycloalkyl- oder $C_3$-$C_8$-Cycloalkoxygruppen, die Aminogruppe, $C_1$-$C_8$-Alkylamino- oder Di-($C_1$-$C_6$)-alkylaminogruppen, Amino-$C_1$-$C_4$-alkylgruppen, $C_1$-$C_6$-Alkylamino-$C_1$-$C_4$-alkyl- oder Di-($C_1$-$C_6$)-alkylamino-$C_1$-$C_4$-alkylgruppen, $C_1$-$C_4$-Alkoxygruppen, $C_1$-$C_4$-Alkylthiogruppen, $C_1$-$C_4$-Alkoxy-$C_1$-$C_4$-alkylgruppen, $C_1$-$C_6$-Alkylthio-$C_1$-$C_4$-alkylgruppen, $C_1$-$C_6$-Alkylcarbonyl- oder $C_1$-$C_6$-Alkylcarbonyloxygruppen, $C_1$-$C_6$-Alkoxycarbonylgruppen, die Hydroxycarbonylgruppe, Hydroxycarbonyl-$C_1$-$C_4$-alkylgruppen, Hydroxycarbonyl-$C_1$-$C_4$-alkoxy- oder Hydroxycarbonyl-$C_1$-$C_4$-alkylthiogruppen, die Hydroxysulfonylgruppe, $C_1$-$C_6$-Alkylsulfinyl-oder $C_1$-$C_6$-Alkylsulfonylgruppen, $C_1$-$C_4$-Alkoxysulfonylgruppen, die Aminosulfonylgruppe, $C_1$-$C_4$-Alkylaminosulfonyl- oder Di-($C_1$-$C_4$)-alkylaminosulfonylgruppen, die Hydrazinogruppe, die bis zu drei $C_1$-$C_4$-Alkylreste tragen kann, die Phenylgruppe, Phenyl-$C_1$-$C_3$-alkylgruppen, die Naphthylgruppe, Naphthyl-$C_1$-$C_3$-alkylgruppen, 5- oder 6-gliedrige Heteroaryl- oder Heteroaryl-$C_1$-$C_3$-alkylgruppen, die Phenoxy- oder Naphthoxygruppe, die Phenylthio- oder Naphthylthiogruppe, die Phenoxycarbonyl- oder Naphthoxycarbonylgruppe, die Phenylcarbonyl- oder Naphthylcarbonylgruppe, die Phenylcarbonyloxy- oder Naphthylcarbonyloxygruppe, Phenyl-$C_1$-$C_3$-alkoxy- oder Naphthyl-$C_1$-$C_3$-alkoxygruppen, 5- oder 6-gliedrige Heteroaryl-$C_1$-$C_3$-alkoxygruppen, die Phenylsulfinyl- oder Naphthylsulfinylgruppe, 5- oder 6-gliedrige Heteroarylsulfinylgruppen, die Phenylsulfonyl- oder Naphthylsulfonylgruppe, 5- oder 6-gliedrige Heteroarylsulfonylgruppen, die Phenoxysulfonyl- oder Naphthoxysulfonylgruppe, 5- oder 6-gliedrige Heteroaryloxysulfonylgruppen, wobei die Aryl- und Heteroarylteile der genannten Substituenten noch bis zu drei der folgenden Substituenten tragen können: Halogen, Cyano, Nitro, $C_1$-$C_4$-Alkyl, $C_1$-$C_4$-Alkoxy, Amino, $C_1$-$C_6$-Alkylamino, Di-($C_1$-$C_6$)-alkylamino oder partiell oder vollständig halogeniertes $C_1$-$C_4$-Alkyl;

X eine $C_1$-$C_6$-Alkylenkette, die noch einen der folgenden Reste tragen kann: Halogen, Hydroxy, Mercapto, $C_1$-$C_4$-Alkyl, $C_1$-$C_4$-Alkoxy, $C_1$-$C_4$-Alkylthio, Di-($C_1$-$C_4$)-alkylamino, $C_1$-$C_4$-Alkoxycarbonyl, Cyano, $C_1$-$C_6$-Alkylcarbonyl, $C_1$-$C_6$-Alkylcarbonyloxy, Phenyl, Naphthyl, 5- oder 6-gliedriges Heteroaryl, Phenyl-($C_1$-$C_3$)-alkyl, Naphthyl-($C_1$-$C_3$)-alkyl oder 5- oder 6-gliedriges Heteroaryl-($C_1$-$C_3$)-alkyl, wobei die Aryl- und Heteroarylteile der letztgenannten sechs Reste noch bis zu 3 Halogenatome oder $C_1$-$C_4$-Alkylgruppen tragen können;

$R^5$ Wasserstoff, Cyano, eine $C_1$-$C_6$-Alkylgruppe, eine $C_2$-$C_6$-Alkenylgruppe, eine $C_1$-$C_4$-Alkoxy-$C_1$-$C_4$-alkylgruppe, eine $C_1$-$C_4$-Alkylthio-$C_1$-$C_4$-alkylgruppe, eine $C_1$-$C_4$-Alkoxycarbonylgruppe, die Acetylgruppe, eine $C_3$-$C_6$-Cycloalkylgruppe, die Benzoyl-, Naphthoyl- oder eine 5- oder 6-gliedrige Heteroaroylgruppe, die Phenyl- oder Naphthylgruppe, eine Phenyl-$C_1$-$C_3$-alkyl- oder Naphthyl-$C_1$-$C_3$-alkylgruppe, eine 5- oder 6-gliedrige Heteroaryl-oder Heteroaryl-$C_1$-$C_3$-alkylgruppe, wobei die Aryl- und Heteroarylteile der letztgenannten sechs Gruppen noch bis zu drei der folgenden Reste tragen können: Halogen, Hydroxy, Cyano, Trifluormethyl, $C_1$-$C_6$-Alkoxy, das unsubstituiert oder partiell oder vollständig halogeniert sein kann, $C_1$-$C_6$-Alkylthio, $C_1$-$C_6$-Alkyl, Di-($C_1$-$C_3$)-alkylamino oder Acetamino;

$R^6$ Wasserstoff, Cyano, eine $C_1$-$C_6$-Alkylgruppe, die unsubstituiert oder partiell oder vollständig halogeniert sein kann, eine $C_1$-$C_4$-Alkoxygruppe, eine $C_1$-$C_4$-Alkoxy-$C_1$-$C_4$-alkylgruppe, eine Diazolylmethyl- oder Triazolylmethylgruppe, eine $C_1$-$C_6$-Alkoxycarbonylgruppe, die Furyl-

oder Tetrahydrofurylgruppe, die Thienylgruppe, eine Dihydropyranyl- oder Dihydrothiopyranylgruppe, die Tetrahydropyranyl- oder Tetrahydrothiopyranylgruppe, oder eine Phenylgruppe, wenn $R^5$ Wasserstoff, Methyl oder Acetyl bedeutet;
oder
zusammen mit dem Rest $R^5$ und dem gemeinsamen C-Atom einen $C_3$-$C_{12}$-Cycloalkylrest, einen 4-Oxocyclohexadienylrest oder einen 5- oder 6-gliedrigen Ring, der gesättigt oder partiell oder vollständig ungesättigt sein kann und ein Sauerstoff- oder Schwefelatom als Heteroatom enthalten kann und/oder der noch bis zu drei $C_1$-$C_3$-Alkyl- oder $C_1$-$C_3$-Alkoxygruppen oder einen anellierten Benzolring tragen kann;

Y, Z Sauerstoff oder Schwefel;

mit der Maßgabe, daß $R^5$ und $R^6$ nicht gleichzeitig Wasserstoff bedeuten, sowie die pflanzenverträglichen Salze derjenigen Verbindungen Ia, bei denen $R^1$, $R^2$, $R^3$, $R^4$ Hydroxycarbonyl, Hydroxycarbonyl-$C_1$-$C_4$-alkyl, Hydroxycarbonyl-$C_1$-$C_4$-alkoxy, Hydroxycarbonyl-$C_1$-$C_4$-alkylthio oder Hydroxysulfonyl bedeuten.

Außerdem betrifft die Erfindung ein Verfahren zur Herstellung der Verbindungen Ia sowie herbizide Mittel, die 2-(4-Heteroaryloxy)- oder 2-(4-Aryloxy)-phenoxyessigsäure-oder -propionsäurederivate und/oder Cyclohexenonderivate als herbizide Wirkstoffe und substituierte 1,8-Naphthalindicarbonsäureimide der Formel Ia, Ib und/oder Ic, sowie die pflanzenverträglichen Salze derjenigen Verbindungen Ia, Ib und/oder Ic, bei denen $R^1$, $R^2$, $R^3$, $R^4$ Hydroxycarbonyl, Hydroxycarbonyl-$C_1$-$C_4$-alkyl, Hydroxycarbonyl-$C_1$-$C_4$-alkoxy, Hydroxycarbonyl-$C_1$-$C_4$-alkylthio oder Hydroxysulfonyl bedeuten und/oder $R^7$ Hydroxycarbonyl-$C_1$-$C_6$-alkyl, Hydroxycarbonyl-$C_1$-$C_6$-alkoxy oder Hydroxysulfonyl-$C_1$-$C_6$-alkyl bedeutet, als Antidots enthalten.

Ferner betrifft die Erfindung Verfahren zur selektiven Bekämpfung von unerwünschtem Pflanzenwuchs mit diesen herbiziden Mitteln.

1,8-Naphthalindicarbonsäureimide, die am Imidstickstoff u.a. eine Gruppe

$$-(CH_2)_n-CO-N<$$

(n = ganze Zahl) tragen können, werden von der JP-A 7337738-R (als Weißmacher) und von der NL-A 7314760 (als Polyamid-Farbstoffe) umfaßt. Verbindungen Ib, die am Stickstoffatom eine Gruppe $-(CH_2)n$-CO-O-H oder $-(CH_2)_n$-CO-O-Alkyl tragen, sind in der Literatur insbesondere als medizinische Wirkstoffe oder als Bleichmittel für Polymere beschrieben (z.B. US-A 4 254 108, JP-A 54 067 035, JP-A 7338 212-R, JP-A 7227 777-R, JP-A 7002 672-R).

Aus der DE-A 27 53 152 sind 1,8-Naphthalincarbonsäureimide, die am Imidstickstoff eine geg. subst. Cyanomethyl-, Carboxymethyl- oder eine acetalisierte Carboxymethylgruppe tragen, als optische Aufheller bekannt.

Herbizide Wirkstoffe aus der Gruppe der 2-(4-Heteroaryloxy)- oder 2-(4-Aryloxy)-phenoxyessigsäurederivate der Formel IV,

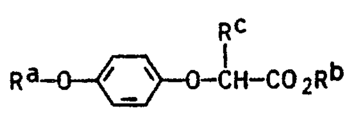

IV

in der die Substituenten folgende Bedeutung haben:

- $R^a$ die Phenylgruppe, die Pyridylgruppe, eine Benzoxazylgruppe, eine Benzthiazylgruppe oder eine Benzpyrazinylgruppe, wobei diese aromatischen Ringsysteme bis zu zwei der folgenden Reste tragen können: Halogen, Nitro, $C_1$-$C_4$-Alkyl, $C_1$-$C_4$-Halogenalkyl und/oder $C_1$-$C_4$-Halogenalkoxy,
- $R^b$ Wasserstoff, eine $C_1$-$C_4$-Alkylgruppe oder das Äquivalent eines pflanzenverträglichen Kations und
- $R^c$ Wasserstoff oder eine Methylgruppe,

sind aus der Literatur, z.B. aus DE-A-22 23 894, DE-A-24 33 067, DE-A-25 76 251, DE-A-30 04 770, BE-A-868 875 und BE-A-858 618 bekannt.

Sie dienen der Bekämpfung von unerwünschten Pflanzen aus der Familie der Gramineen. Die Verträglichkeit dieser Substanzen für Kulturpflanzen variiert jedoch zwischen kommerziell acceptabel und unverträglich, je nach Substituenten und Aufwandmenge.

Ebenso verhält es sich mit Cyclohexenonderivaten der Formel V,

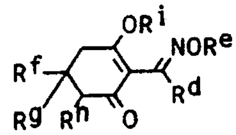

V

in welcher die Substituenten folgende Bedeutung haben:

$R^d$ eine $C_1$-$C_4$-Alkylgruppe;

$R^e$ eine $C_1$-$C_4$-Alkylgruppe, eine $C_3$-$C_4$-Alkenylgruppe, eine $C_3$-$C_4$-Alkinylgruppe oder eine $C_3$-$C_4$-Halogenalkenylgruppe;
einen -($C_1$-$C_4$-Alkyl)-phenyl oder -($C_1$-$C_4$-Alkenyl)-phenylrest, wobei der Alkyl- und Alkenylteil noch bis zu 3 $C_1$-$C_3$-Alkylgruppen und/oder Halogenatome und der Phenylteil bis zu 5 Halogenatome oder einen Benzyloxycarbonyl- oder Phenylrest und/oder bis zu 3 der folgenden Substituenten tragen kann: Nitro, Cyano, $C_1$-$C_4$-Alkyl, $C_1$-$C_4$-Alkoxy, $C_1$-$C_4$-Alkylthio, partiell oder vollständig halogeniertes $C_1$-$C_4$-Alkyl, partiell oder vollständig halogeniertes $C_1$-$C_4$-Alkoxy, Carboxyl oder $C_1$-$C_4$-Alkoxycarbonyl;
die Thienylgruppe, die noch ein Halogenatom tragen kann;

$R^f$ eine $C_1$-$C_4$-Alkylgruppe, welche ein- oder zweifach durch $C_1$-$C_4$-Alkylthio oder $C_1$-$C_4$-Alkoxy substituiert sein kann;
ein 5- oder 6-gliedriges gesättigtes oder einfach ungesättigtes Ringsystem, welches neben Kohlenstoffgliedern ein Sauerstoff-, ein Schwefelatom oder eine Sulfoxid- oder Sulfongruppe enthalten kann, und wobei dieser Ring bis zu drei der folgenden Reste tragen kann: Hydroxy, Halogen, $C_1$-$C_4$-Alkyl, $C_1$-$C_4$-Halogenalkyl, $C_1$-$C_4$-Alkoxy und/oder $C_1$-$C_4$-Alkylthio;
einen 10-gliedrigen gesättigten oder einfach ungesättigten Heterocyclus, weloher zwei nicht benachbarte Sauerstoffatome oder Schwefelatome enthält und der durch bis zu drei $C_1$-$C_4$-Alkylgruppen und/oder Methoxygruppen substituiert sein kann;
eine Phenylgruppe, eine Pyridylgruppe, eine Thiazolylgruppe, eine Pyrazolylgruppe, eine Pyrrolylgruppe oder eine Isoxazolylgruppe, wobei diese Gruppen bis zu drei der folgenden Gruppen tragen können: $C_1$-$C_4$-Alkyl, $C_1$-$C_4$-Halogenalkyl, $C_1$-$C_4$-Alkoxy, $C_1$-$C_4$-Alkylthio, $C_3$-$C_6$-Alkenyloxy, $C_3$-$C_6$-Alkinyloxy, $C_1$-$C_4$-Alkoxy-$C_1$-$C_3$-alkyl, $C_1$-$C_4$-Dialkoxy-$C_1$-$C_3$-alkyl, Formyl, Halogen und/oder Benzoylamino

$R^g$ Wasserstoff, Hydroxy oder, wenn $R^f$ die Bedeutung $C_1$-$C_6$-Alkylgruppe hat, eine $C_1$-$C_6$-Alkylgruppe;

$R^h$ Wasserstoff, eine Cyanogruppe, ein Halogenatom, eine $C_1$-$C_4$-Alkoxycarbonylgruppe oder eine $C_1$-$C_4$-Alkylketoximgruppe und

$R^i$ Wasserstoff oder das Äquivalent eines umweltverträglichen Kations.

Sie sind in der Literatur (z.B. EP-A 228 598, EP-A 230 235, EP-A 238 021, EP-A 368 227, US-A 4 432 786, DE-A 24 39 104) ebenfalls als Herbizide beschrieben und dienen vorwiegend zur Bekämpfung unerwünschter Gräser in dicotylen Kulturen und in Gräsern, die nicht zur Familie der Gramineen zählen. Je nach Struktur der Substituenten und Dosis der Anwendung können Verbindungen aus dieser Gruppe auch zur selektiven Bekämpfung von unerwünschten Gräsern in Gramineen Kulturen wie Weizen und Reis eingesetzt werden.

Cyclohexenonderivate der Formel V, in denen $R^e$ für einen unsubstituierten oder substituierten Alkyl- oder Alkenyl-, z.B. Butyl- oder Butenylphenylrest steht, können in an sich bekannter Weise aus schon bekannten Derivaten der Formel VIII (EP-A-80 301, EP-A-125 094, EP-A 142 741, USA 4 249 937, EP-A 137 174 und EP-A 177 913) und den entsprechenden Hydroxylaminen der Formel IX (Houben-Weyl, 10/1, S. 1181 ff) hergestellt werden (EP-A 169 521).

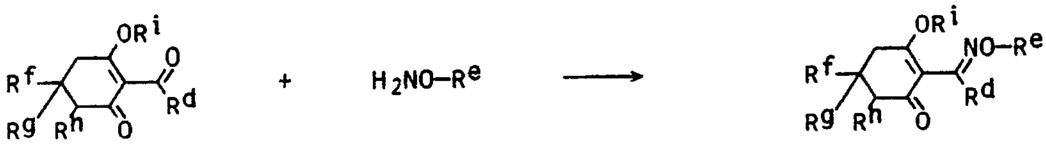

VIII IX V

Zweckmäßig führt man die Umsetzung in heterogener Phase in einem Lösungsmittel, bei einer ausreichenden Temperatur unterhalb von etwa 80 °C, in Gegenwart einer Base durch und verwendet das Hydroxylamin IX in Form seines Ammoniumsalzes.

Geeignete Basen sind z. B. Carbonate, Hydrogencarbonate, Acetate, Alkoholate oder Oxide von Alkali- oder Erdalkalimetallen, insbesondere Natriumhydroxid, Kaliumhydroxid, Magnesiumoxid, Calciumoxid. Außerdem können organische Basen wie Pyridin oder tertiäre Amine Verwendung finden. Die Base wird beispielsweise in einer Menge von 0,5 bis 2 Mol-Äquivalent bezogen auf die Ammoniumverbindung zugesetzt.

Als Lösungsmittel eignen sich beispielsweise Dimethylsulfoxid; Alkohole wie Methanol, Ethanol und Isopropanol; aromatische Kohlenwasserstoffe wie Benzol und Toluol; chlorierte Kohlenwasserstoffe wie Chloroform und Dichlorethan; aliphatische Kohlenwasserstoffe wie Hexan und Cyclohexan; Ester wie Essigsäureethylester und Ether wie Diethylether, Dioxan und Tetrahydrofuran. Vorzugsweise führt man die Umsetzung in Methanol mit Natriumhydrogencarbonat als Base durch.

Die Reaktion ist nach wenigen Stunden beendet. Die Zielverbindung kann z.B. durch Einengen der Mischung, Verteilung des Rückstandes in Methylenchlorid/Wasser und Abdestillieren des Lösungsmittels unter vermindertem Druck isoliert werden.

Man kann für diese Umsetzung aber auch unmittelbar die freie Hydroxylaminbase, z.B. in Form einer wäßrigen Lösung, verwenden; je nach verwendetem Lösungsmittel für die Verbindung VIII erhält man ein ein- oder zweiphasiges Reaktionsgemisch.

Geeignete Lösungsmittel für diese Variante sind beispielsweise Alkohole wie Methanol, Ethanol, Isopropanol und Cyclohexanol; aliphatische und aromatische, gegebenenfalls chlorierte Kohlenwasserstoffe wie Hexan, Cyclohexan, Methylenchlorid, Toluol und Dichlorethan; Ester wie Essigsäureethylester; Nitrile wie Acetonitril und cyclische Ether wie Dioxan und Tetrahydrofuran.

Alkalimetallsalze der Verbindungen V können durch Behandeln der 3-Hydroxyverbindungen mit Natrium- oder Kaliumhydroxid bzw. -alkoholat in wäßriger Lösung oder in einem organischen Lösungsmittel wie Methanol, Ethanol, Aceton und Toluol erhalten werden.

Andere Metallsalze wie Mangan-, Kupfer-, Zink-, Eisen-, Calcium-, Magnesium- und Bariumsalze können aus den Natriumsalzen in üblicher Weise hergestellt werden, ebenso Ammonium- und Phosphoniumsalze mittels Ammoniak, Ammonium-, Phosphonium-, Sulfonium- oder Sulfoxoniumhydroxiden.

Die Verbindungen des Typs VIII können z.B. aus den entsprechenden Cyclohexan-1,3-dionen der Formel X

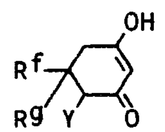

X,

in der

Y Wasserstoff oder Methoxycarbonyl bedeutet und

$R^g$ Wasserstoff bedeutet

nach bekannten Methoden (Tetrahedron Lett., 2491 (1975)) hergestellt werden.

Es ist auch möglich, die Verbindungen der Formel VIII über die Zwischenstufe der Enolester herzustellen, die bei der Umsetzung von Verbindungen der Formel X mit Säurechloriden in Gegenwart von Basen anfallen und anschließend mit bestimmten Imidazol- oder Pyridinderivaten umgelagert werden (JP-OS 79/063 052).

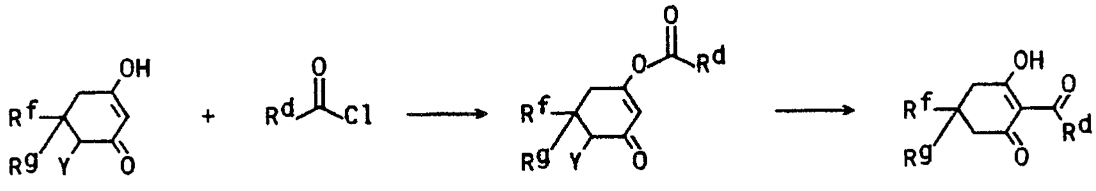

X

VIII

Zu den Verbindungen der Formel X gelangt man über eine Reihe bekannter Verfahrensschritte

ausgehend von bekannten Vorprodukten.

Die Synthese der Hydroxylamine IX, in denen $R^e$ für einen ggf. substituierten Phenylbutylrest steht, erfolgt gemäß dem nachstehenden Reaktionsschema beispielsweise über

A) die Alkylierung von cyclischen Hydroxyimiden XI mit geeigneten Phenylbutylhalogeniden und anschließende Schutzgruppenabspaltung beispielsweise mit Hydrazin oder Ethanolamin analog Beispielen aus EP-A-244 786 bzw. Houben-Weyl, Methoden der organischen Chemie, Band X/1, Seite 1152ff.

B) Hydrierung von N-4-Phenylbutenyloxyphthalimiden, deren Herstellung in DE-A 38 38 310 beschrieben ist, mittels geeigneten Katalysatoren wie z.B. Palladium auf Aktivkohle, in geeigneten inerten Lösungsmitteln wie z.B. Methanol, Tetrahydrofuran, Dioxan und anschließende Schutzgruppenabspaltung wie voranstehend beschrieben.

Vorteilhaft wird die Hydrierung bei Temperaturen von 20°C bis zum Siedepunkt des Lösungsmittels, insbesondere bei Raumtemperatur nach den dafür üblichen Techniken bei Atmosphärendruck, über- oder Unterdruck durchgeführt. Bevorzugt ist ein Druckbereich von 1 bis 10, insbesondere 1 bis 2 bar.

Reaktionsschema:

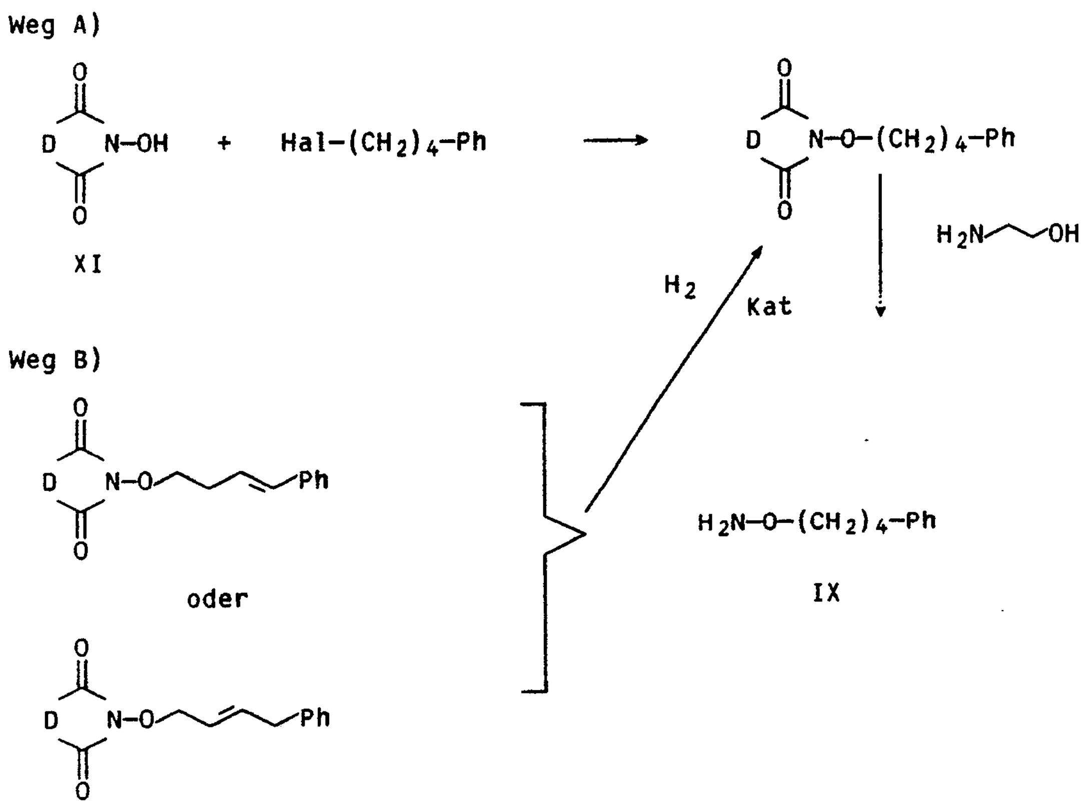


Ph = ggf. substituierter Phenylrest

Als cyclische Hydroxyimide XI kommen beispielsweise folgende Substanzen in Betracht:

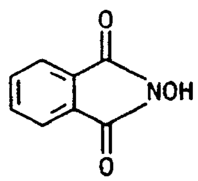
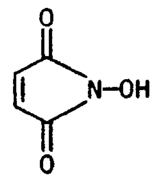
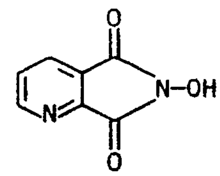

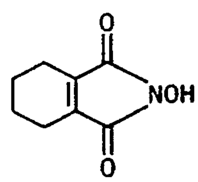
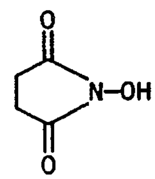
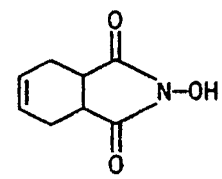

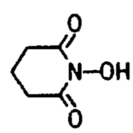

Die Synthese der Hydroxylamine IX, in denen $R^e$ für einen unsubstituierten oder substituierten Butenylphenylrest steht, wobei der nachstehend mit Ph abgekürzte Phenylrest seinerseits substituiert oder unsubstituiert sein kann, erfolgt gemäß dem nachstehenden Reaktionsschema ausgehend von Anilinderivaten durch Diazotierung und anschließende Kupplung des Diazoniumsalzes mit einem entsprechend substituierten Butadien XII. Das so erhaltene Gemisch aus XIIIa und XIIIb wird mit einem cyclischen Hydroxyimid XI gekoppelt und das erhaltene geschützte Hydroxylaminderivat XIV mit 2-Aminoethanol zum freien Hydroxylamin IX gespalten:

Weg C)

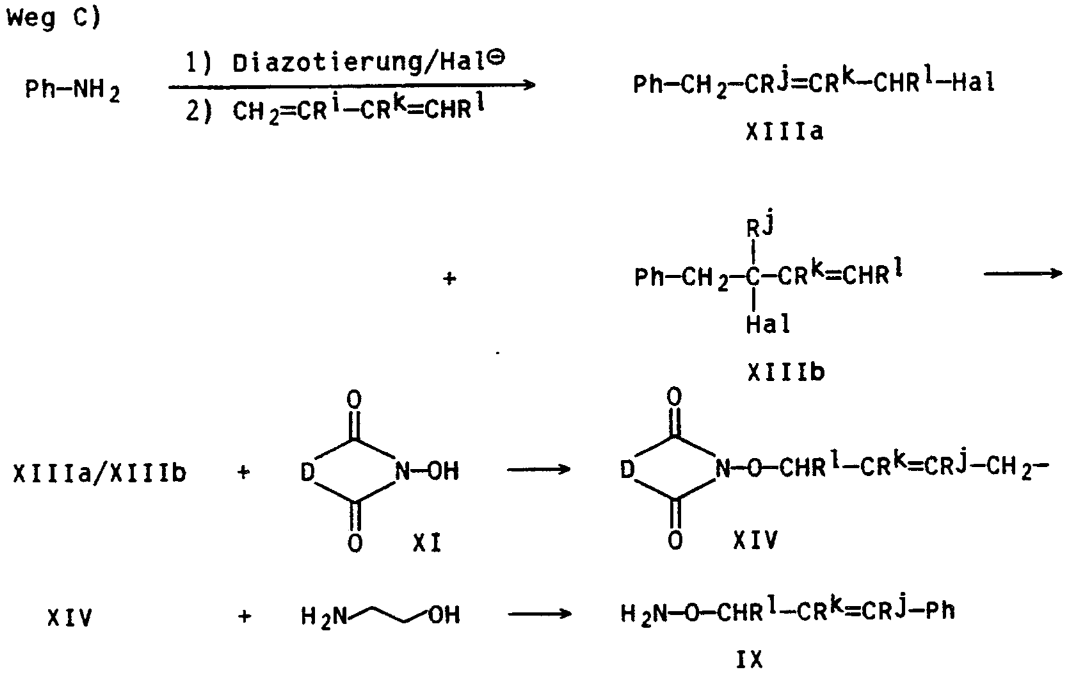


XIIIa, XIIIb, XI, XIV, IX

Die Reste $R^j$, $R^k$ und $R^l$ bedeuten hierbei unabhängig voneinander Wasserstoff, $C_1$-$C_3$-Alkylgruppen und/oder Halogenatome. Hal bedeutet Halogenatom, vorzugsweise Chloratom.

Die zur obigen Synthese der Hydroxylamine der Formel IX benötigten Halogenide XIIIa lassen sich nach literaturbekannten Verfahren im Gemisch mit XIIIb herstellen, beispielsweise durch die Umsetzung von Diazoniumsalzen aromatischer und heteroaromatischer Aniline mit Dienen. Die Anwendungsbreite der Reaktion ist in Organic Reactions 11 (1960) 189 bzw. 24 (1976) 225 abgehandelt.

Bei der Kopplung der isomeren Halogenide XIIIa und XIIIb an ein cyclisches Hydroxyimid der Formel XI entstehen ausschließlich die cyclischen Imidether der Formel XIV, die nach Abspaltung der Schutzgruppe am Stickstoff die Hydroxylamine IX liefern.

Die Umsetzung mit einem Hydroxyimid XI (Weg A und C) erfolgt in Gegenwart eines säurebindenden Mittels und eines Lösungsmittels. Aus Kostengründen kommt als Hydroxyimid XI bevorzugt Hydroxyphthalimid zum Einsatz.

Als säurebindende Mittel eignen sich Alkalimetallcarbonate wie Kalium- oder Natriumcarbonat, Alkalimetallhydrogencarbonate wie Kalium- und Natriumhydrogencarbonat, tertiäre Amine wie Trimethyl- und Triethylamin und basische Heterocyclen wie Pyridin. Aus Kostengründen kommen bevorzugt Kalium- und Natriumcarbonat in Betracht.

Als Lösungsmittel eignen sich aprotisch dipolare organische Lösungsmittel wie z.B. Dimethylformamid, Dimethylsulfoxid und/oder Sulfolan.

Ferner ist auch eine Alkylierung unter Phasentransferbedingungen möglich. Als organische Lösungsmittel werden hierbei mit Wasser nicht mischbare Verbindungen wie Kohlenwasserstoffe oder Chlorkohlenwasserstoffe eingesetzt. Als Phasentransferkatalysatoren eignen sich quartäre Ammonium- und Phosphoniumsalze.

Die Spaltung der cyclischen Imidether XIV gelingt analog einem in EP-A 244 786 beschriebenen Verfahren mit Alkanolaminen. Die Hydroxylamine IX können nach diesem Verfahren als freie Basen oder nach Fällung mit Säuren als Salze isoliert werden. Gut kristallisierende Salze erhält man durch Umsetzung der Basen mit Oxalsäure.

Der vorliegenden Erfindung lag nun die Aufgabe zugrunde, Verbindungen zu finden, welche die Nachteile, die bei der Verwendung der obengenannten Herbizide der Formeln IV und V bestehen, zumindest so stark zu verringern, daß der Ernteertrag der Kulturpflanzen nicht mehr oder nicht nennenswert herabgesetzt wird.

Entsprechend der Aufgabe wurden die eingangs definierten substituierten 1,8-Naphthalindicarbonsäureimide Ia, Ib und Ic gefunden.

Weiterhin wurden Verfahren zur Herstellung der Verbindungen Ia sowie Verfahren zur kombinierten Behandlung von Pflanzenkulturen mit den antidotisch wirkenden Verbindungen Ia und/oder Ib und/oder Ic einerseits und den Herbiziden IV und/oder V andererseits gefunden, wobei es unerheblich ist, ob der herbizide Wirkstoff und die antidotische Verbindung gemeinsam oder getrennt formuliert und ausgebracht werden bzw. bei getrennter Ausbringung, in welcher Reihenfolge herbizider Wirkstoff und Antidot appliziert werden.

Derivate Ia, Ib und Ic mit sauren Endgruppen können in Form ihrer landwirtschaftlich brauchbaren Salze vorliegen. Bevorzugt werden dabei die Alkalimetallsalze, insbesondere das Kalium- oder Natriumsalz, Erdalkalimetallsalze, insbesondere das Calcium-, Magnesium- oder Bariumsalz, Mangan-, Kupfer-, Zink- oder Eisensalze sowie Ammonium-, Phosphonium-, Sulfonium- oder Sulfoxoniumsalze, beispielsweise Ammoniumsalze, Alkylammoniumsalze wie Isopropylammoniumsalze, Tetraalkylammoniumsalze, Benzyltriarylammoniumsalze, Trialkylsulfoniumsalze oder Trialkylsulfoxoniumsalze.

Die 1,8-Naphthalindicarbonsäureimide Ia, Ib und Ic sind z.B. nach folgenden Methoden erhältlich:

a) Die Verbindungen Ib und Ic sind bekannt und können nach bekannten Verfahren dargestellt werden [z.B. Prog.Clin.Biol.Res. (Enzym.Mol.Biol. Carbonyl Metab.) 232, 353 (1987); 3.Med.Chem. 29, 2384 (1986); Egypt. J. Chem. 24, 127 (1981); Ind.J.Chem., Sect. B, 21B, 1106 (1982); Egypt. J. Chem. 25, 445 (1982), sowie die dort angeführte Literatur].

Im allgemeinen wird ein 1,8-Naphthalindicarbonsäureanhydrid oder ein 1,4, 5, 8-Naphthalintetracarbonsäuredianhydrid der Formel VIa bzw. VIb mit einer Aminoverbindung VII kondensiert:

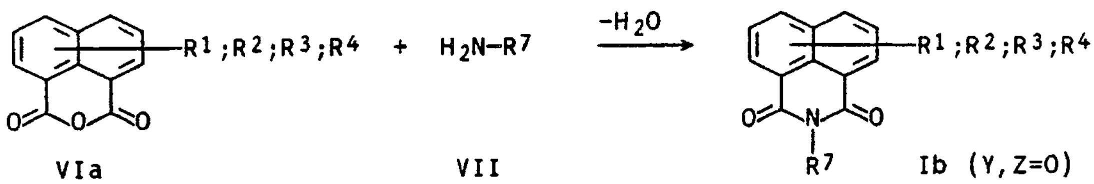


VIa VII Ib (Y,Z=O)

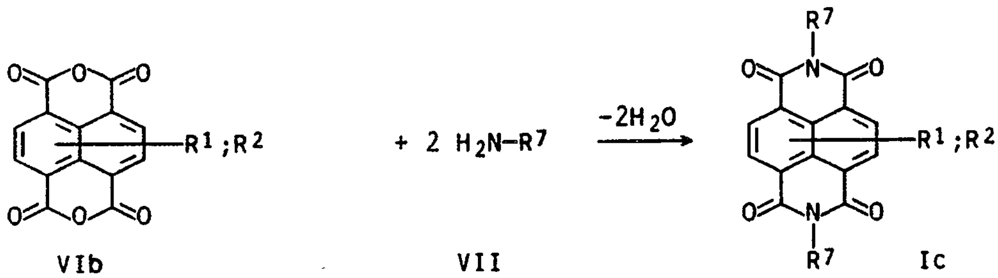


VIb VII Ic

Üblicherweise setzt man die Ausgangsstoffe VI und VII in stöchiometrischem Verhältnis ein, jedoch kann ein Überschuß der einen oder anderen Komponente in Einzelfällen vorteilhaft sein.

Die Reaktionstemperatur liegt im allgemeinen bei 0 bis 250 °C, vorteilhaft im Siedebereich des Lösungsmittels.

Die so dargestellten 1,8-Naphthalindicarbonsäureimide Ib und Ic können nach üblichen Methoden in weitere Derivate Ib bzw. Ic überführt werden, so daß das Substitutionsmuster in einem weiten Bereich variiert werden kann.

b) Die erfindungsgemäßen Verbindungen Ia können zweckmäßigerweise durch Umsetzung eines Säurehalogenids der Formel II mit einem Oxim III in Gegenwart einer Base erhalten werden:

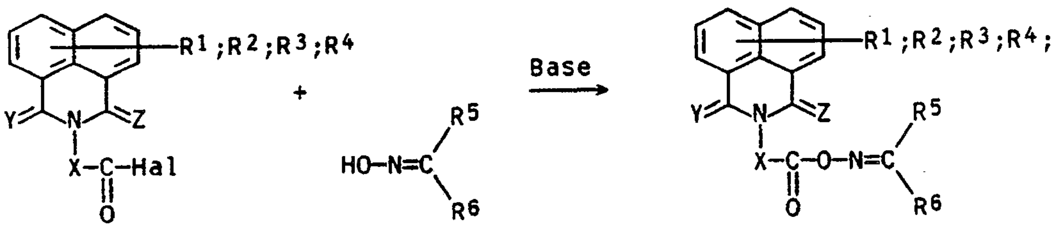


II III Ia

Als Lösungsmittel eignen sich inerte aliphatische oder aromatische, gewünschtenfalls chlorierte Kohlenwasserstoffe wie Pyridin, Toluol, Chlorbenzol und Methylenchlorid sowie Ether wie Diethylether, Methyl-tert.-butylether, Tetrahydrofuran und Dioxan oder geeignete Gemische der genannten Solventien.

Als Base verwendet man zweckmäßigerweise Verbindungen wie Pyridin, 4-Dimethylaminopyridin, Diazabicycloundecen (DBU), Diazabicyclooctan (DABCO), tertiäre aliphatische Amine wie Triethylamin oder (bei der Herstellungsvariante nach Schotten-Baumann) wäßrige Alkalilauge oder Alkalicarbonatlösung.

Üblicherweise setzt man die Ausgangsstoffe II und III in stöchiometrischem Verhältnis ein, jedoch kann ein Überschuß der einen oder anderen Komponente in Einzelfällen vorteilhaft sein.

Die Umsetzung kann bei Normal-, über- oder Unterdruck nach den dafür üblichen Techniken durchgeführt werden. Die Reaktionstemperatur liegt im allgemeinen bei (-10) bis 150 °C, insbesondere bei 10 bis 100 °C. Vorteilhaft arbeitet man bei Normaldruck im Siedebereich des Lösungsmittels.

Die Aufarbeitung geschieht bei Verwendung von Pyridin als Base und Lösungsmittel im allgemeinen durch Eingießen der Reaktionsmischung in Wasser, im Falle von mit Wasser nicht mischbaren Lösungsmitteln dagegen im allgemeinen durch Extraktion mit Wasser, waschen mit verdünnten Mineralsäuren und Entsäuern. Die Reinigung der Rohprodukte erfolgt z.B. durch Umkristallisation, durch Umfällung oder durch Chromatographie.

Die Oxime III sind bekannt oder nach bekannten Verfahren erhältlich.

Bei ungleichen Resten $R^5$ und $R^6$ liegen die Oxime III meist als Isomerengemisch der syn-/anti-Isomeren vor, so daß die Endprodukte Ia ebenfalls als Isomerengemisch anfallen können.

Als Säurehalogenide II können die Fluoride, Chloride oder Bromide verwendet werden. Besonders bevorzugt werden die Säurechloride. Man erhält sie vorteilhaft durch Halogenierung der entsprechenden Carbonsäuren mit einem Halogenierungsmittel wie Thionylchlorid, Phosphortrichlorid oder Phosphorpentachlorid. Die Carbonsäuren sind bekannt oder können analog den Verbindungen Ib synthetisiert werden.

Zur Darstellung der Verbindungen Ia können neben den Säurehalogeniden II auch andere aktivierte Carbonsäurederivate wie Carbonsäureanhydride oder Carbonsäureimidazolide eingesetzt werden.

Im Hinblick auf die bestimmungsgemäße Verwendung der Verbindungen Ia, Ib und Ic als pflanzenschützende Mittel kommen als Substituenten folgende Reste in Betracht:

$R^1$-$R^4$

- Wasserstoff, Hydroxy, Mercapto, Cyano, Thiocyanato, Nitro;
- Halogen, insbesondere Fluor, Chlor und Brom;
- $C_1$-$C_6$-Alkylgruppen, oder $C_1$-$C_6$-Halogenalkylgruppen, verzweigt oder unverzweigt, insbesondere $C_1$-$C_4$-Alkyl- oder $C_1$-$C_4$-Halogenalkylgruppen wie Methyl, Ethyl, tert.-Butyl und Trifluormethyl, Pentafluorethyl und 2-Chlor-1,1,2-trifluorethyl;
- $C_2$-$C_6$-Alkenylgruppen, insbesondere $C_2$-$C_4$-Alkenylgruppen wie Ethenyl, 2-Propenyl, 3-Butenyl und 3-Butenyl;
- $C_2$-$C_6$-Alkinylgruppen, insbesondere $C_2$-$C_4$-Alkinylgruppen, z.B. 2-Propinyl;
- $C_3$-$C_6$-Cycloalkyl- oder Cycloalkoxygruppen, insbesondere Cyclopropyl, Cyclopentyl, Cyclohexyl und Cyclohexyloxy;
- Amino-, $C_1$-$C_8$-Alkylamino- oder Di-($C_1$-$C_6$)-alkylaminogruppen, insbesondere Amino, $C_1$-$C_4$-Alkylamino- oder Di($C_1$-$C_4$)-alkylaminogruppen, z.B. Methylamino, tert.-Butylamino, Dimethylamino, Methylethylamino und Diethylamino;
- Amino-($C_1$-$C_4$)-alkyl-, $C_1$-$C_6$-Alkylamino-($C_1$-$C_4$)-alkyl- oder Di($C_1$-$C_6$)alkylamino($C_1$-$C_4$)-alkylgruppen, insbesondere Amino-($C_1$-$C_2$)-alkyl-, $C_1$-$c_4$-Alkylamino-($C_1$-$C_2$)-alkyl- oder Di($C_1$-$C_4$)-alkylamino-($C_1$-$C_2$)-alkylgruppen, z.B. Aminomethyl, Methylaminomethyl, Dimethylaminomethyl und Methylethylaminomethyl;
- $C_1$-$C_4$-Alkoxy- oder $C_1$-$C_4$-Alkylthiogruppen, z.B. Methoxy, Ethoxy, tert.-Butoxy, Methylthio und Ethylthio;
- $C_1$-$C_4$-Alkoxy-$C_1$-$C_4$-alkyl- oder $C_1$-$C_4$-Alkylthio-$C_1$-$C_4$-alkylgruppen, insbesondere $C_1$-$C_3$-Alkoxy-$C_1$-$C_2$-alkyl- oder $C_1$-$C_3$-Alkylthio-$C_1$-$C_2$-alkylgruppen, z.B. Methoxymethyl, Methoxyethyl, Ethoxymethyl, tert.-Butoxymethyl, Methylthiomethyl und Ethylthiomethyl;
- $C_1$-$C_6$-Alkylcarbonyl- oder $C_1$-$C_6$-Alkylcarbonyloxygruppen, insbesondere $C_1$-$C_4$-Alkylcarbonyl- oder $C_1$-$C_4$-Alkylcarbonyloxygruppen wie Methylcarbonyl, n-Butylcarbonyl, Methylcarbonyloxy und tert.-Butylcarbonyloxy;
- $C_1$-$C_6$-Alkoxycarbonylgruppen, insbesondere $C_1$-$C_4$-Alkoxycarbonylgruppen wie Methoxycarbonyl, Ethoxycarbonyl und tert.-Butoxycarbonyl;
- Hydroxycarbonyl- oder Hydroxycarbonyl-$C_1$-$C_3$-alkylgruppen, insbesondere Hydroxycarbonyl, Hydroxycarbonylmethyl und Hydroxycarbonylethyl;
- Hydroxycarbonyl-$C_1$-$C_4$-alkoxy- oder Hydroxycarbonyl-$C_1$-$C_4$-alkylthiogruppen, insbesondere Hydroxycarbonylmethoxy, Hydroxycarbonylethoxy, Hydroxycarbonylmethylthio und Hydroxycarbonylethylthio;
- die Hydroxysulfonylgruppe;
- $C_1$-$C_6$-Alkylsulfinyl- oder $C_1$-$C_6$-Alkylsulfonylgruppen, insbesondere $C_1$-$C_4$-Alkylsulfinyl- oder $C_1$-$C_4$-Alkylsulfonylgruppen, Methylsulfinyl, tert.-Butylsulfinyl, Methylsulfonyl und tert.-Butylsulfonyl;
- $C_1$-$C_4$-Alkoxysulfonylgruppen, insbesondere Methoxysulfonyl, Ethoxysulfonyl und tert.-Butoxysulfonyl;
- die Aminosulfonylgruppe, $C_1$-$C_4$-Alkylaminosulfonyl- oder Di($C_1$-$C_4$)-alkylaminosulfonylgruppen, insbesondere Methylaminosulfonyl, Ethylaminosulfonyl, tert.-Butylaminosulfonyl, Methylethylaminosulfonyl, Dimethylaminosulfonyl und Diethylaminosulfonyl;
- die Hydrazinogruppe, die bis zu 3 $C_1$-$C_4$-Alkylreste tragen kann, insbesondere Hydrazino;
- die Phenylgruppe oder Phenyl-$C_1$-$C_3$-alkylgruppen, wobei die Arylteile bis zu 3 der folgenden Substituenten tragen können: Halogen, Cyano, Nitro, $C_1$-$C_4$-Alkyl wie Methyl und Ethyl, $C_1$-$C_4$-Alkoxy wie Methoxy, Amino, $C_1$-$C_4$-Alkylamino wie Methylamino, Di-($C_1$-$C_6$)-alkylamino wie Dimethylamino oder partiell oder vollständig halogeniertes $C_1$-$C_4$-Alkyl wie Trichlormethyl und Trifluormethyl, insbesondere Phenyl, Benzyl, Phenylethyl und Phenyl-n-propyl;

- die Naphthylgruppe oder Naphthyl-$C_1$-$C_3$-alkylgruppen, wobei die Arylteile bis zu 3 der Substituenten wie für die Phenylgruppe genannt tragen können, insbesondere Naphthyl, Naphth-1-ylmethyl und Naphth-1-ylethyl;
- 5- oder 6-gliedrige Heteroaryl- oder Heteroaryl-$C_1$-$C_3$-alkylgruppen, deren Heteroarylteile bis zu 3 der Substituenten wie für die Phenylgruppe vorstehend genannt tragen können, insbesondere 2-Pyrrolyl, 2-Thienyl, 3-Furanyl und 2-Pyridyl;
- die Phenyloxy- oder Naphthyloxygruppe, die bis zu 3 der Substituenten wie für die Phenylgruppe vorstehend genannt tragen können, insbesondere Phenoxy und Naphthoxy;
- die Phenylthio- oder Naphthylthiogruppe, die jede bis zu 3 der Substituenten wie für die Phenylgruppe vorstehend genannt tragen kann, insbesondere Phenylthio und Naphthylthio;
- die Phenoxycarbonyl- oder Naphthoxycarbonylgruppe, die jede bis zu 3 der Substituenten wie für die Phenylgruppe vorstehend genannt tragen kann, insbesondere Phenoxycarbonyl und Naphthoxycarbonyl;
- die Phenylcarbonyl- oder Naphthylcarbonylgruppe, die jede bis zu 3 der Substituenten wie für die Phenylgruppe vorstehend genannt tragen kann, insbesondere Phenylcarbonyl und Naphthylcarbonyl;
- die Phenylcarbonyloxy- oder Naphthylcarbonyloxygruppe, die jede bis zu 3 der Substituenten wie für die Phenylgruppe vorstehend genannt tragen kann, insbesondere Phenylcarbonyloxy und und Naphthylcarbonyloxy;
- Phenyl-$C_1$-$C_3$-alkoxy- oder Naphthyl-$C_1$-$C_3$-alkoxygruppen, die bis zu 3 der Substituenten wie für die Phenylgruppe vorstehend genannt tragen können, insbesondere Phenyl-$C_1$-$C_2$-alkoxy-oder Naphthyl-$C_1$-$C_3$-alkoxygruppen, z.B. Phenylmethoxy und Naphth-1-ylmethoxy;
- 5- oder 6-gliedrige Heteroaryl-$C_1$-$C_3$-alkoxygruppen, die bis zu 3 der Substituenten wie für die Phenylgruppe vorstehend genannt tragen können, insbesondere Pyrrolylmethyloxy;
- die Phenylsulfinyl- oder Naphthylsulfinylgruppe, die jede bis zu 3 der Substituenten wie für die Phenylgruppe vorstehend genannt tragen kann, insbesondere Phenylsulfinyl und Naphthylsulfinyl;
- 5- oder 6-gliedrige Heteroarylsulfinyl- oder Heteroarylsulfonylgruppen, die bis zu 3 der Substituenten wie für die Phenylgruppe vorstehend genannt tragen können;
- die Phenylsulfonyl- oder Naphthylsulfonylgruppe, die jede bis zu 3 der Substituenten wie für die Phenylgruppe vorstehend genannt tragen kann, insbesondere Phenylsulfonyl und Naphthylsulfonyl;
- die Phenoxysulfonyl- oder Naphthoxysulfonylgruppe, die jede bis zu 3 der Substituenten wie für die Phenylgruppe vorstehend genannt tragen kann, insbesondere Phenoxysulfonyl und Naphthoxysulfonyl;
- 5- oder 6-gliedrige Heteroaryloxysulfonylgruppen, die bis zu 3 der Substituenten wie für die Phenylgruppe vorstehend genannt tragen können;

X

- eine $C_1$-$C_6$-Alkylenkette, die noch einen der folgenden Reste tragen kann: Halogen wie Fluor, Chlor und Brom, Hydroxy, Mercapto, $C_1$-$C_4$-Alkyl wie Methyl und tert.-Butyl, $C_1$-$C_4$-Alkoxy, $C_1$-$C_4$-Alkylthio, Di-($C_1$-$C_4$)-alkylamino, $C_1$-$C_4$-Alkoxycarbonyl, Cyano, $C_1$-$C_6$-Alkylcarbonyl, $C_1$-$C_6$-Alkylcarbonyloxy, Phenyl, Naphthyl, 5- oder 6-gliedriges Heteroaryl, phenyl-($C_1$-$C_3$)-alkyl, Naphthyl-($C_1$-$C_3$)-alkyl oder 5- oder 6-gliedriges Heteroaryl-($C_1$-$C_3$)-alkyl, wobei die Aryl- und Heteroarylteile der letztgenannten 6 Reste noch bis zu 3 Halogenatome oder $C_1$-$C_4$-Alkylgruppen tragen können;

$R^5$

- Wasserstoff, Cyano;
- eine $C_1$-$C_6$-Alkylgruppe, verzweigt oder unverzweigt, insbesondere eine $C_1$-$C_4$-Alkylgruppe wie Methyl, Ethyl, n-Propyl, Isopropyl, n-Butyl und tert.-Butyl;
- eine $C_2$-$C_6$-Alkenylgruppe, insbesondere eine $C_2$-$C_4$-Alkenylgruppe wie Ethenyl und 3-Butenyl;
- eine $C_1$-$C_4$-Alkoxy-$C_1$-$C_4$-alkyl- oder $C_1$-$C_4$-Alkylthio-$C_1$-$C_4$-alkylgruppe, insbesondere eine $C_1$-$C_3$-Alkoxy-$C_1$-$C_2$-alkyl- oder $C_1$-$C_3$-Alkylthio-$C_1$-$C_2$-alkylgruppe, z.B. Methoxymethyl, Ethoxyethyl und Methylthiomethyl;
- eine $C_1$-$C_4$-Alkoxycarbonylgruppe, insbesondere Methoxycarbonyl und Ethoxycarbonyl;
- eine Acetylgruppe,
- eine $C_3$-$C_6$-Cycloalkylgruppe, insbesondere Cyclopropyl, Cyclopentyl und Cyclohexyl,
- die Benzoyl-, Naphthoyl- oder eine 5- oder 6-gliedrige Heteroaroylgruppe, insbesondere Benzoyl, Naphthoyl, Pyridylcarbonyl und Thienylcarbonyl;
- die Phenyl- oder Naphthylgruppe, die jeweils bis zu 3 der folgenden Reste tragen kann: Halogen wie

Fluor, Chlor und Brom, Hydroxy, Cyano, Trifluormethyl, verzweigtes oder unverzweigtes $C_1$-$C_6$-Alkyl, $C_1$-$C_6$-Alkoxy, partiell oder vollständig substituiertes $C_1$-$C_6$-Alkoxy, $C_1$-$C_6$-Alkylthio, Di-($C_1$-$C_3$)-alkylamino oder Acetamino, insbesondere Phenyl und Naphthyl;

- eine Phenyl-$C_1$-$C_3$-alkyl- oder Naphthyl-$C_1$-$C_3$-alkylgruppe, die bis zu 3 der Reste wie bei der Phenylgruppe genannt tragen können, insbesondere Benzyl, Phenethyl und Naphth-1-ylmethyl;
- eine 5- oder 6-gliedrige Heteroaryl- oder Heteroaryl-$C_1$-$C_3$-alkylgruppe, die bis zu 3 der Reste wie bei der Phenylgruppe vorstehend genannt tragen kann, insbesondere 2-Pyrrolyl, 2-Thienyl, 3-Furanyl und 2-Pyridyl;

$R^6$

- Wasserstoff, Cyano;
- eine $C_1$-$C_6$-Alkylgruppe oder $C_1$-$C_6$-Halogenalkylgruppe, verzweigt oder unverzweigt, insbesondere eine $C_1$-$C_4$-Alkyl- oder $C_1$-$C_4$-Halogenalkylgruppe, z.B. Methyl, Ethyl, n-Propyl, Isopropyl, n-Butyl, tert.-Butyl, Trifluormethyl, Pentafluorethyl und 2-Chlor-1, 1, 2-trifluorethyl;
- eine $C_1$-$C_4$-Alkoxygruppe, insbesondere Methoxy, Ethoxy und tert.-Butoxy;
- eine $C_1$-$C_4$-Alkoxy-$C_1$-$C_4$-alkylgruppe, insbesondere eine $C_1$-$C_3$-Alkoxy-$C_1$-$C_2$-alkylgruppe, z.B. Methoxymethyl, Ethoxymethyl und Methoxyethyl;
- eine Diazolylmethyl- oder Triazolylmethylgruppe, insbesondere 1,3-Diazol-1-ylmethyl, 1,2-Diazol-1-ylmethyl, 1,2,4-Triazol-1-ylmethyl und 1,3,4-Triazol-1-ylmethyl;
- eine $C_1$-$C_6$-Alkoxycarbonylgruppe, insbesondere eine $C_1$-$C_4$-Alkoxycarbonylgruppe, z.B. Methoxycarbonyl, Ethoxycarbonyl und tert.-Butoxycarbonyl;
- die Furyl- oder Tetrahydrofurylgruppe;
- die Tetrahydropyranyl- und Tetrahydrothiopyranylgruppe;
- die Thienylgruppe;
- eine Dihydropyranyl- und Dihydrothiopyranylgruppe;
- die Phenylgruppe, wenn $R^5$ Wasserstoff, Methyl oder Acetyl bedeutet,

mit der Maßgabe, daß $R^5$ und $R^6$ nicht gleichzeitig Wasserstoff bedeuten;

oder

zusammen mit dem Rest $R^5$ und dem gemeinsamen C-Atom

- einen $C_3$-$C_{12}$-Cycloalkylrest, insbesondere Cyclopropyl, Cyclopentyl, Cyclohexyl, Cycloheptyl und Cyclododecyl;
- einen 5- oder 6-gliedrigen Ring, der gesättigt oder partiell oder vollständig ungesättigt sein kann und ein Sauerstoff- oder Schwefelatom als Heteroatom enthalten kann, und/oder der noch bis zu 3 $C_1$-$C_3$-Alkyl- oder $C_1$-$C_3$-Alkoxygruppen oder einen anellierten Benzolring tragen kann, insbesondere 3,3,5-Trimethylcyclohexyliden, 3-Methyl-cyclopentyliden, 3-Methylcyclohexyliden, 3-Methylcyclopent-2-enyliden, Cyclohex-2-enyliden, 2-Methoxy-cyclohex-2-enyliden, 2,6-Dimethylpyranyliden-4, 2,6-Dimethylthiopyranyliden-4, Tetrahydropyranyliden-4, Tetrahydrothiopyranyliden-4, 2,3-Benzocyclopentyliden-1 und 2,3-Benzocyclohexyliden-2;

$R^7$

- Wasserstoff, Hydroxyl, Cyano;
- eine $C_1$-$C_6$-Alkyl- oder $C_1$-$C_6$-Halogenalkylgruppe, verzweigt oder unverzweigt, insbesondere eine $C_1$-$C_4$-Alkyl- oder $C_1$-$C_4$-Halogenalkylgruppe wie Methyl, Ethyl, n-Propyl, tert.-Butyl, Trifluormethyl, Pentafluorethyl und 2-Chlor-1,1,2-trifluorethyl;
- eine $C_1$-$C_6$-Hydroxyalkylgruppe, insbesondere eine $C_1$-$C_4$-Hydroxyalkylgruppe wie Hydroxymethyl, Hydroxyethyl, 4-Hydroxy-n-but-1-yl und 2-Hydroxy-1, 1-dimethylethyl;
- eine $C_2$-$C_6$-Alkenylgruppe, insbesondere eine $C_2$-$C_4$-Alkenylgruppe wie Ethenyl, 2-Propen-1-yl und But-3-enyl;
- eine $C_1$-$C_4$-Alkoxygruppe, insbesondere Methoxy, Ethoxy, Isopropoxy und n-Butoxy;
- eine $C_1$-$C_4$-Alkoxy-$C_1$-$C_4$-alkylgruppe, insbesondere eine $C_1$-$C_3$-Alkoxy-$C_1$-$C_2$-alkylgruppen, z.B. Methoxymethyl, Methoxyethyl, Ethoxymethyl und Ethoxyethyl;
- eine $C_1$-$C_4$-Alkylthio-$C_1$-$C_4$-alkylgruppe, insbesondere eine $C_1$-$C_3$-Alkylthio-$C_1$-$C_2$-alkylgruppe wie Methylthiomethyl, Methylthioethyl und Ethylthiomethyl;
- eine Amino-, $C_1$-$C_4$-Alkylamino- oder Di-($C_1$-$C_4$)-alkylaminogruppe, insbesondere Amino, Methylamino, tert.-Butylamino, Dimethylamino, Diethylamino und Methylethylamino;
- eine $C_3$-$C_8$-Cycloalkyl- oder $C_3$-$C_8$-Cycloalkoxygruppe, insbesondere Cyclopropyl, Cyclopentyl, Cyclohexyl, Cyclopentoxy und Cyclohexoxy;
- eine Acylgruppe;

- eine $C_1$-$C_4$-Alkoxycarbonyl-$C_1$-$C_6$-alkylgruppe, insbesondere eine $C_1$-$C_3$-Alkoxycarbonyl-$C_1$-$C_2$-alkylgruppe, z.B. Methoxycarbonylmethyl, Ethoxycarbonylmethyl, n-Propoxycarbonylmethyl und Ethoxycarbonylethyl;
- eine Aminocarbonyl-$C_1$-$C_6$-alkyl-, $C_1$-$C_4$-Alkylaminocarbonyl-$C_1$-$C_6$-alkyl- oder Di-($C_1$-$C_4$)-alkylaminocarbonyl-$C_1$-$C_6$-alkylgruppe, insbesondere eine Aminocarbonyl-$C_1$-$C_2$-alkyl-, $C_1$-$C_3$-Alkylaminocarbonyl-$C_1$-$C_2$-alkyl- oder Di-($C_1$-$C_3$)-alkylaminocarbonyl-$C_1$-$C_2$-alkylgruppe, z.B. Aminocarbonylmethyl, Methylaminocarbonylmethyl, Dimethylaminocarbonylmethyl, Aminocarbonylethyl und Diethylaminocarbonylethyl;
- eine $C_1$-$C_4$-Alkoxyaminocarbonyl-$C_1$-$C_6$-alkyl- oder N-($C_1$-$C_4$-Alkoxy)-N-($C_1$-$C_4$-alkyl)-aminocarbonyl-$C_1$-$C_6$-alkylgruppe, insbesondere eine $C_1$-$C_3$-Alkoxyaminocarbonyl-$C_1$-$C_2$-alkyl-oder N-($C_1$-$C_3$-Alkoxy)-N-($C_1$-$C_3$-alkyl)-aminocarbonyl-$C_1$-$C_2$-alkylgruppe, z.B. Methoxyaminocarbonylmethyl, N-Methoxy-N-methyl-aminocarbonylmethyl und N-Ethoxy-N-ethyl-aminocarbonylethyl;
- eine $C_1$-$C_4$-Alkylthiocarbonyl-$C_1$-$C_6$-alkylgruppe, insbesondere eine $C_1$-$C_3$-Alkylthiocarbonyl-$C_1$-$C_2$-alkylgruppe, z.B. Methylthiocarbonylmethyl und Ethylthiocarbonylethyl;
- eine $C_1$-$C_4$-Alkoxycarbonyl- oder eine $C_1$-$C_4$-Alkylcarbonyloxygruppe, insbesondere Methoxycarbonyl;
- eine $C_1$-$C_4$-Alkoxysulfonyl-$C_1$-$C_6$-alkylgruppe, insbesondere eine $C_1$-$C_3$-Alkoxysulfonyl-$C_1$-$C_2$-alkylgruppe, z.B. Methoxysulfonylmethyl, Methoxysulfonylethyl und n-Propoxysulfonylethyl;
- eine Aminosulfonyl-$C_1$-$C_6$-alkyl, $C_1$-$C_4$-Alkylaminosulfonyl-$C_1$-$C_6$-alkyl- oder Di($C_1$-$C_4$)-alkylaminosulfonyl-$C_1$-$C_6$-alkylgruppe, insbesondere eine Aminosulfonyl-$C_1$-$C_2$-alkyl-, $C_1$-$C_3$-Alkylaminosulfonyl-$C_1$-$C_2$-alkyl oder Di-($C_1$-$C_3$)-alkylaminosulfonyl-$C_1$-$C_2$-alkylgruppe, z.B. Aminosulfonylmethyl, Dimethylaminosulfonylmethyl und Diethylaminosulfonylethyl;
- eine Hydroxycarbonyl-$C_1$-$C_6$-alkyl- oder eine Hydroxycarbonyl-$C_1$-$C_6$-alkoxygruppe, insbesondere eine Hydroxycarbonyl-$C_1$-$C_2$-alkyl- oder Hydroxycarbonyl-$C_1$-$C_2$-alkoxygruppe wie Hydroxycarbonylmethyl, Hydroxycarbonylethyl und Hydroxycarbonylmethoxy;
- eine Hydroxysulfonyl-$C_1$-$C_6$-alkylgruppe, insbesondere eine Hydroxysulfonyl-$C_1$-$C_2$-alkylgruppe wie Hydroxysulfonylmethyl und Hydroxysulfonylethyl;
- die Phenyl- oder eine Phenyl-$C_1$-$C_4$-alkylgruppe, wobei der Aromat jeweils noch bis zu 3 Halogenatome oder $C_1$-$C_4$-Alkylgruppen oder einen der folgenden Reste tragen kann: Nitro, Cyano, Hydroxycarbonyl, Amino, $C_1$-$C_4$-Alkylamino, Di-($C_1$-$C_4$)-Alkylamino, $C_1$-$C_4$-Alkoxy oder partiell oder vollständig halogeniertes $C_1$-$C_4$-Alkyl, insbesondere Phenyl, Benzyl, Phenethyl und 4-Phenylbutyl;
- die Naphthyl- oder eine Naphthyl-$C_1$-$C_4$-alkylgruppe, wobei der Aromat jeweils noch bis zu 3 Halogenatome oder $C_1$-$C_4$-Alkylgruppen oder einen der Reste wie bei der vorstehenden Phenylgruppe genannt tragen kann, insbesondere Naphthyl, Naphth-1-ylmethyl und Naphth-1-yl-2-ethyl;
- eine 5- oder 6-gliedrige Heteroaryl- oder Heteroaryl-$C_1$-$C_4$-alkylgruppe, wobei der Aromat jeweils noch bis zu 3 Halogenatome oder $C_1$-$C_4$-Alkylgruppen oder einen der Reste wie bei der vorstehenden Phenylgruppe genannt tragen kann, insbesondere Pyrrolyl, Thienyl, Furanyl, Pyridyl, 2-Pyridylmethyl, 3-Pyridylmethyl und 4-Pyridylmethyl;
- die Phenoxy- oder eine Phenyl-$C_1$-$C_4$-alkoxygruppe, wobei der Aromat jeweils noch bis zu 3 Halogenatome oder $C_1$-$C_4$-Alkylgruppen oder einen der Reste wie bei der vorstehenden Phenylgruppe genannt tragen kann, insbesondere Phenoxy, Benzyloxy, 2-Phenylethoxy und 4-Phenylbutoxy;
- die Naphthoxy- oder eine Naphthyl-$C_1$-$C_4$-alkoxygruppe, wobei der Aromat- jeweils noch bis zu 3 Halogenatome oder $C_1$-$C_4$-Alkylgruppen oder einen der Reste wie bei der vorstehenden Phenylgruppe genannt tragen kann, insbesondere Naphthoxy, Naphth-1-ylmethoxy und 4-(Naphth-1-yl)-butoxy;
- die Benzoylgruppe, die noch bis zu 3 Halogenatome oder $C_1$-$C_4$-Alkylgruppen oder einen der Reste wie bei der vorstehenden Phenylgruppe genannt tragen kann,
- eine Phenoxycarbonyl-$C_1$-$C_4$-alkyl- oder Naphthoxycarbonyl-$C_1$-$C_4$-alkylgruppe, wobei der Aromat jeweils noch bis zu 3 Halogenatome oder $C_1$-$C_4$-Alkylgruppen oder einen der Reste wie bei der vorstehenden Phenylgruppe genannt tragen kann, insbesondere Phenoxycarbonylmethyl, 4-(Phenoxycarbonyl)-butyl, 1-Naphthoxycarbonyl-methyl und 4-(1-Naphthoxycarbonyl)-butyl;
- eine 5- oder 6-gliedrige Heteroaryloxycarbonyl-$C_1$-$C_4$-alkylgruppe, wobei der Aromat jeweils noch bis zu 3 Halogenatome oder $C_1$-$C_4$-Alkylgruppen oder einen der Reste wie bei der vorstehenden Phenylgruppe genannt tragen kann, insbesondere 3-Pyridyloxycarbonyl und Thienyloxycarbonyl;
- eine Gruppe $-(CH_2)_n-COO-NR^8R^9$, wobei n eine ganze Zahl von 1 bis 6 und $R^8$, $R^9$ $C_1$-$C_3$-Alkyl, insbesondere Methyl oder Ethyl, oder $C_1$-$C_3$-Alkylcarbonyl, insbesondere Methylcarbonyl, bedeutet, insbesondere $-(CH_2)_2-COO-N(CH_3)(CO-CH_3)$,
- eine Gruppe $-(CH_2)n-COO-NR^8R^9$, wobei n eine ganze Zahl von 1 bis 6 bedeutet und die Reste $R^8$ und $R^9$ mit dem gemeinsamen Stickstoffatom einen 5- oder 6-gliedrigen Ring bilden, insbesondere Pyrrolidin-1-yloxycarbonyl und 2(pyrrolidin-1-yl-oxycarbonyl)ethyl.

Besonders gut geeignete Verbindungen Ia, Ib und Ic sind den Tabellen 1, 2 und 3 zu entnehmen.

Spezielle Beispiele für herbizide (Heteroaryloxy)- bzw. Aryloxy-phenoxyessigsäurederivate der Formel IV, deren Kulturpflanzenverträglichkeit durch substituierte 1,8-Naphthalindicarbonsäureimide der Formel Ia, Ib und/oder Ic verbessert werden kann, sind in der folgenden Tabelle A aufgeführt.

Tabelle A

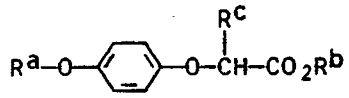

IV

| Nr. | $R^a$ | $R^b$ | $R^c$ | Literatur |
|---|---|---|---|---|
| IV.1 | 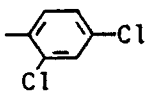 | $CH_3$ | $CH_3$ | DE-A 22 23 894 |
| IV.2 | 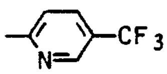 | $n-C_4H_9$ | $CH_3$ | BE-A 868 875 |
| IV.3 | 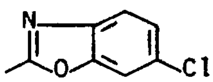 | $C_2H_5$ | $CH_3$ | BE-A 858 618 |
| IV.4 | 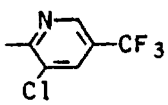 | $CH_3$ | $CH_3$ | BE-A 868 875 |
| IV.5 | 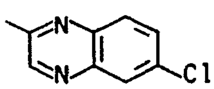 | $C_2H_5$ | $CH_3$ | DE-A 30 04 770 |

Spezielle Beispiele für herbizide Cyclohexenone der Formel V, deren Kulturpflanzenverträglichkeit durch substituierte 1,8-Naphthalindicarbonsäureimide der Formel Ia, Ib und/oder Ic verbessert werden kann, sind in den folgenden Tabellen B und C aufgeführt.

Tabelle B

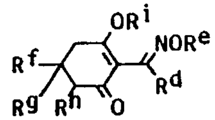 V

| Nr. | $R^d$ | $R^e$ | $R^f$ | $R^g$ | $R^h$ | $R^i$ | Literatur |
|---|---|---|---|---|---|---|---|
| V.1 | $C_3H_7$ | $CH_2CH{=}CH_2$ | $CH_3$ | $CH_3$ | $CO_2CH_3$ | Na | DE-A 2 439 104 |
| V.2 | $C_3H_7$ | $CH_2CH_3$ | $CH_2CH(CH_3)SCH_2CH_3$ | H | H | H | DE-A 2 822 304 |
| V.3 | $C_2H_5$ | $CH_2CH{=}CHCl$ | $CH_2CH(CH_3)SCH_2CH_3$ | H | H | H | US-A 4 440 566 |
| V.4 | $C_3H_7$ | $CH_2CH{=}CHCl$ | $CH_2CH(CH_3)SCH_2CH_3$ | H | H | H | US-A 4 440 566 |
| V.5 | $C_3H_7$ | $C_2H_5$ | 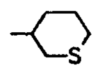 | H | H | H | EP-A 71 707 |
| V.6 | $C_2H_5$ | $C_2H_5$ | 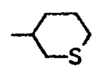 | H | H | H | EP-A 71 707 |
| V.7 | $CH_3$ | $CH_2CH{=}CHCH_3$ | 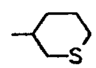 | H | H | H | EP-A 71 707 |
| V.8 | $C_3H_7$ | $C_2H_5$ | 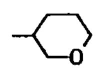 | H | H | H | EP-A 71 707 |
| V.9 | $C_2H_5$ | $CH_2CH{=}CHCl$ | 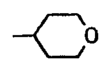 | H | H | H | EP-A 142 741 |

Tabelle B (Fortsetzung)

| Nr. | $R^d$ | $R^e$ | $R^f$ | $R^g$ | $R^h$ | $R^i$ | Literatur |
|---|---|---|---|---|---|---|---|
| V.10 | $C_3H_7$ | $C_2H_5$ | 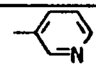 | H | H | H | EP-A 66 195 |
| V.11 | $C_2H_5$ | $C_2H_5$ | 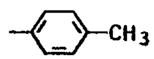 | H | H | H | DE-A 24 39 104 |
| V.12 | $C_2H_5$ | $CH_2CH{=}CHCH_3$ | 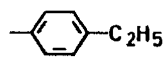 | H | H | H | deutsche Anmeldung P 38 08 072.9 |
| V.13 | $C_2H_5$ | $C_2H_5$ | 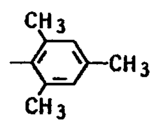 | H | H | H | EP-A 880 301 |
| V.14 | $C_3H_7$ | $CH_2CH{=}CHCl$ | 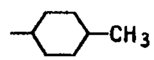 | H | H | H | EP-A 88 299 |
| V.15 | $C_3H_7$ | $CH_2CH{=}CHCH_3$ | 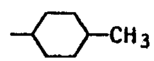 | H | H | H | EP-A 88 299 |
| V.16 | $C_2H_5$ | $CH_2CH{=}CHCH_3$ | 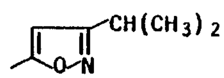 | H | H | H | EP-A 238 021 |
| V.17 | $C_3H_7$ | $CH_2CH{=}CHCH_3$ | 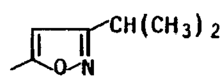 | H | H | H | EP-A 238 021 |
| V.18 | $C_2H_5$ | $CH_2CH{=}CHCl$ | 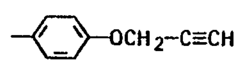 | H | H | H | EP-A 137 174 |

Tabelle B (Fortsetzung)

| Nr. | $R^d$ | $R^e$ | $R^f$ | $R^g$ | $R^h$ | $R^i$ | Literatur |
|---|---|---|---|---|---|---|---|
| V.19 | $C_3H_7$ | $C_2H_5$ | 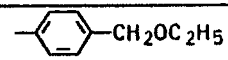 | H | H | H | EP-A 2 137 200 |
| V.20 | $C_3H_7$ | $C_2H_5$ | 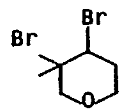 | H | H | H | EP-A 230 235 |
| V.21 | $C_3H_7$ | $CH_2CH{=}CHCl$ | 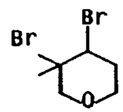 | H | H | H | EP-A 230 235 |
| V.22 | $C_3H_7$ | $CH_2CH{=}CHCl$ | 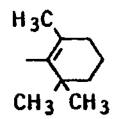 | H | H | H | EP-A 46 860 |
| V.23 | $C_3H_7$ | $C_2H_5$ | 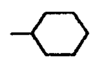 | H | H | H | JP-A 540 191 945 |
| V.24 | $C_3H_7$ | $C_2H_5$ | 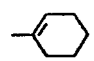 | H | H | H | EP-A 46 860 |
| V.25 | $CH_3$ | $CH_2CH{=}CHCl$ | 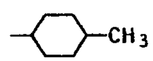 | H | H | H | EP-A 88 299 |
| V.26 | $C_3H_7$ | $C_2H_5$ | 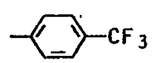 | H | H | K | EP-A 137 174 |
| V.27 | $C_2H_5$ | $CH_2CH{=}CHCl$ | 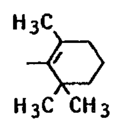 | H | H | H | EP-A 46 860 |

Tabelle B (Fortsetzung)

| Nr. | $R^d$ | $R^e$ | $R^f$ | $R^g$ | $R^h$ | $R^i$ | Literatur |
|---|---|---|---|---|---|---|---|
| V.28 | $C_3H_7$ | $CH_2CH{=}CHCH_3$ | 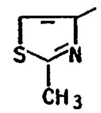 | H | H | H | EP-A 125 094 |
| V.29 | $C_3H_7$ | $CH_2CH{=}CHCl$ | 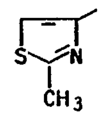 | H | H | H | EP-A 125 094 |
| V.30 | $C_3H_7$ | $C_2H_5$ | 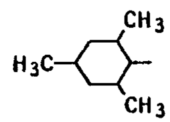 | H | H | H | EP-A 88 299 |
| V.31 | $C_3H_7$ | $CH_2CH{=}CH_2$ | 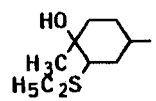 | H | H | H | EP-A 228 598 |
| V.32 | $C_2H_5$ | $C_2H_5$ | 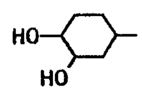 | H | H | H | EP-A 228 598 |
| V.33 | $C_3H_7$ | $C_2H_5$ | 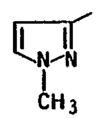 | H | H | H | EP-A 66 195 |

Tabelle B (Fortsetzung)

| Nr. | $R^d$ | $R^e$ | $R^f$ | $R^g$ | $R^h$ | $R^i$ | Literatur |
|---|---|---|---|---|---|---|---|
| V.34 | $C_3H_7$ | $CH_2CH=CHCl$ | 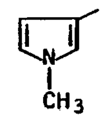 | H | H | H | EP-A 66195 |
| V.35 | $C_3H_7$ | $CH_2CH=CH_2$ | 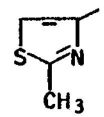 | H | H | H | EP-A 125 094 |
| V.36 | $C_3H_7$ | $C_3H_7$ | $CH(SCH_2CH_3)_2$ | H | H | H | EP-A 230 260 |
| V.37 | $C_3H_7$ | $C_2H_5$ | 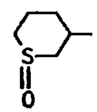 | H | H | H | EP-A 115 808 |
| V.38 | $C_3H_7$ | $C_2H_5$ | 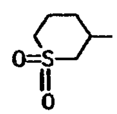 | H | H | H | EP-A 115 808 |
| V.39 | $C_3H_7$ | $C_2H_5$ | $CH_3$ | $CH_3$ | $C(CH_3)=NOCH_3$ | H | EP-A 172 551 |
| V.40 | $C_3H_7$ | $CH_2CH=CH_2$ | 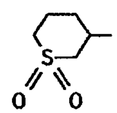 | OH | H | H | Proceedings Brit. Crop-Protection Conference - weeds 1985 Vol.1 S. 93-98 |

Tabelle B (Fortsetzung)

| Nr. | $R^d$ | $R^e$ | $R^f$ | $R^g$ | $R^h$ | $R^i$ | Literatur |
|---|---|---|---|---|---|---|---|
| V.41 | $C_2H_5$ | 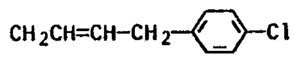 | 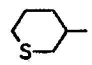 | H | H | H | EP-A 89 120 558.2 |
| V.42 | $C_2H_5$ | 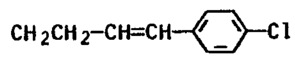 | 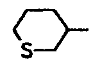 | H | H | H | EP-A 89 120 558.2 |
| V.43 | $C_2H_5$ | 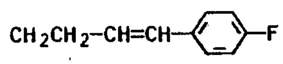 | 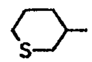 | H | H | H | EP-A 89 120 558.2 |
| V.44 | $n-C_3H_7$ | 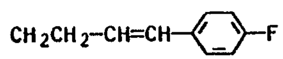 | 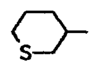 | H | H | H | EP-A 89 120 558.2 |
| V.45 | $n-C_2H_5$ | 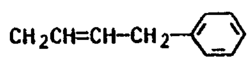 | 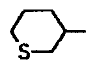 | H | H | H | |
| V.46 | $n-C_3H_7$ | 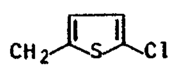 | 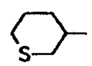 | H | H | H | EP-A 177 913 |
| V.47 | $C_2H_5$ | 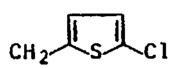 | 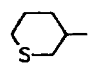 | H | H | H | EP-A 177 913 |
| V.48 | $C_2H_5$ | 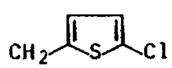 | 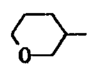 | H | H | H | EP-A 177 913 |

Tabelle B (Fortsetzung)

| Nr. | $R^d$ | $R^e$ | $R^f$ | $R^g$ | $R^h$ | $R^i$ | Literatur |
|---|---|---|---|---|---|---|---|
| V.49 | $n-C_3H_7$ | 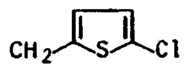 | 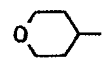 | H | H | H | EP-A 177 913 |
| V.50 | $n-C_3H_7$ | 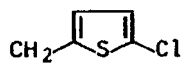 | 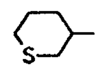 | H | H | H | EP-A 177 913 |
| V.51 | $CH_3$ | 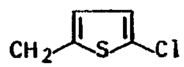 | 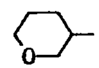 | H | H | H | EP-A 177 913 |
| V.52 | $C_2H_5$ | 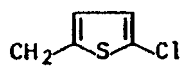 | 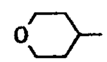 | H | H | H | EP-A 177 913 |

Tabelle C

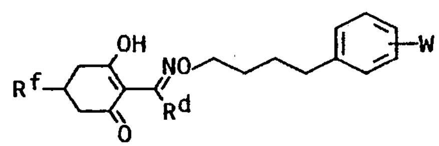

V

| Nr. | $R^d$ | $R^f$ | W | physik. Daten ($^1$H-NMR* [δ in ppm]) |
|---|---|---|---|---|
| V.53 | Ethyl | Tetrahydropyran-3-yl | 4-Fluor | 2.9 (breit, 2H); 4.1 (breit, 2H) |
| V.54 | n-Propyl | Tetrahydropyran-3-yl | 4-Fluor | 2.9 (t, 2H); 4.05 (breit, 2H) |
| V.55 | Ethyl | Tetrahydrothiopyran-3-yl | 4-Fluor | 2.9 (t, 2H); 4.05 (breit, 2H) |
| V.56 | n-Propyl | Tetrahydrothiopyran-3-yl | 4-Fluor | 2.9 (t, 2H); 4.05 (breit, 2H) |
| V.57 | Ethyl | Tetrahydropyran-4-yl | 4-Fluor | 4.05 (breit, 2H) |
| V.58 | n-Propyl | Tetrahydropyran-4-yl | 4-Fluor | 4.05 (breit, 2H) |
| V.59 | Ethyl | Tetrahydropyran-3-yl | 4-Cl | 2.9 (t, 2H); 4.05 (breit, 2H) |
| V.60 | n-Propyl | Tetrahydropyran-3-yl | 4-Cl | 2.9 (t, 2H); 4.05 (breit, 2H) |
| V.61 | Ethyl | Tetrahydropyran-4-yl | 4-Cl | 2.9 (t, 2H); 4.05 (breit, 2H) |
| V.62 | n-Propyl | Tetrahydropyran-4-yl | 4-Cl | 2.9 (breit, 2H); 4.05 (breit, 2H) |
| V.63 | Ethyl | Tetrahydrothiopyran-3-yl | 4-Cl | 4.05 (breit, 2H) |
| V.64 | n-Propyl | Tetrahydrothiopyran-3-yl | 4-Cl | 4.05 (breit, 2H) |

* ausgewählte Signale

Herbizide Wirkstoffe und antidotisch wirkende Verbindungen können gemeinsam oder getrennt nach dem Auflaufen auf die Blätter und Sprosse der Kulturpflanzen und der unerwünschten Gräser ausgebracht werden. Bevorzugt wird das antidotisch wirkende Mittel gleichzeitig mit dem herbiziden Wirkstoff ausgebracht. Auch eine getrennte Ausbringung, wobei das Antidot zuerst und anschließend der herbizide Wirkstoff auf das Feld gebracht werden, ist möglich. Herbizider Wirkstoff und Antidot können hierbei als Spritzmittel in suspendierbarer, emulgierbarer oder löslicher Form gemeinsam oder getrennt formuliert vorliegen.

Denkbar ist auch eine Behandlung der Kulturpflanzensamen mit dem Antidot vor der Aussaat. Der herbizide Wirkstoff wird dann allein in der üblichen Weise appliziert.

Für herbizide (Heteroaryloxy)- bzw. Aryloxyphenoxyessigsäurederivate werden unterschiedliche Mengen einer antidotisch wirkenden Verbindung benötigt, wenn das Herbizid in verschiedenen Kulturen eingesetzt wird. Die Mengenverhältnisse sind in breiten Bereichen variabel. Sie sind ebenfalls abhängig von der Struktur der (Heteroaryloxy)- bzw. Aryloxyphenoxyessigsäurederivate und der jeweiligen Zielkultur. Geeignete Anteilverhältnisse herbizider Wirkstoffe: antidotisch wirkender Verbindung liegen zwischen 1 : 10 bis 1 : 0,01; vorzugsweise zwischen 1 : 4 bis 1 : 0,1 Gew.-Teile.

Für das gleiche Cyclohexenonderivat werden unterschiedliche Mengen einer antidotisch wirkenden Verbindung benötigt, wenn das Cyclohexenonderivat in verschiedenen Kulturen eingesetzt wird. Die Mengenverhältnisse, in denen ein Cyclohexenonderivat und ein 1,8-Naphthalindicarbonsäureimid Ia und/oder Ib eingesetzt werden, sind in breiten Bereichen variabel. Sie sind abhängig von der Struktur des Cyclohexenonderivats, des 1,8-Naphthalindicarbonsäureimids Ia und/oder Ib und der jeweiligen Kultur. Geeignete Anteilverhältnisse herbizider Wirkstoff: antidotisch wirkender Verbindung liegen zwischen 1:10 bis 1:0,01; vorzugsweise 1:4 bis 1:0,25 Gew.-Teile.

Die neuen herbiziden Mittel können neben dem substituierten 1,8-Naphthalindicarbonsäureimid Ia und/oder Ib und/oder Ic als Antidot und dem Herbizid aus der Gruppe der (Heteroaryloxy)- bzw. Aryloxyphenoxyessigsäuren IV bzw. der Cyclohexenone V weitere herbizide oder wachstumsregulierende Wirkstoffe anderer chemischer Struktur enthalten, wobei der antagonistische Effekt erhalten bleibt.

Die erfindungsgemäßen Mittel bzw. bei getrennter Ausbringung die herbiziden Wirkstoffe oder das Antidot werden beispielsweise in Form von direkt versprühbaren Lösungen, Pulvern, Suspensionen, auch hochprozentige wäßrige, ölige oder sonstige Suspensionen, oder Dispersionen, Emulsionen, öldispersionen, Pasten, Stäubemitteln, Streumitteln oder Granulaten durch Versprühen, Vernebeln, Verstäuben, Verstreuen oder Gießen angewendet. Die Anwendungsformen richten sich ganz nach den Verwendungszwecken.

Zur Herstellung von direkt versprühbaren Lösungen, Emulsionen, Pasten und Ölsdispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische oder aromatische Kohlenwasserstoffe, z.B. Benzol, Toluol, xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon und Wasser, in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können herbizider Wirkstoff und/oder Antidot als solche oder in einem Öl oder Lösungsmittel gelöst, mittels Netz-, Haft-, Dispergier- oder Emulgiermittel in Wasser homogenisiert werden. Es können aber auch aus herbizidem Wirkstoff und/oder Antidot Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatierten Fettalkoholglykolethern, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol-, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolylglykoletheracetal, Sorbitester, Lignin-Sulfitablaugen und Methylcellulose in Betracht.

Pulver, Streu- und Stäubmittel können durch Mischen oder gemeinsames Vermahlen von herbizidem Wirkstoff und/oder Antidot mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Die Formulierungen enthalten zwischen 0,1 und 95 Gew.-% an herbizidem Wirkstoff und Antidot,

vorzugsweise zwischen 0,5 und 90 Gew.-%. Die Aufwandmengen an herbizidem Wirkstoff betragen 0,2 bis 5 kg/ha.

Die vorliegende Erfindung wird durch die nachstehend beschriebenen Beispiele weiter erläutert.

Herstellungsbeispiele

Beispiel 1

N-(2 Propylenimino-oxycarbonylmethyl)-1,8-naphthalindicarbonsäureimid

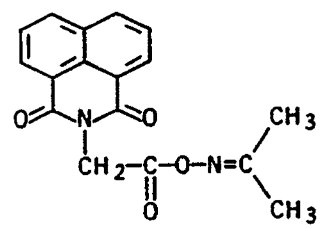

Stufe 1.1: Herstellung des Säurechlorides

Eine Mischung aus 12,8 g (50 mmol) N-Hydroxycarbonylmethyl-1,8-naphthalindicarbonsäureimid, 9,9 g (47,5 mmol) Phosphorpentachlorid und 20 ml $POCl_3$ wurde unter Rühren langsam auf 75°C erhitzt und noch 3 h lang bei dieser Temperatur gerührt. Anschließend wurde das Reaktionsgemisch bis zur Trockene eingeengt und das Rohprodukt ohne Reinigung in der folgenden Stufe weiterverarbeitet.

Stufe 1.2: Kondensation des Säurechlorides mit Acetonoxim

Das in Stufe 1.1 erhaltene Carbonsäurechlorid wurde portionsweise bei 0°C in eine Lösung aus 4,0 g (55 mmol) Acetonoxim und 50 ml Pyridin eingetragen, wonach die resultierende Suspension 12 h bei 20°C weitergerührt wurde. Anschließend wurde das Reaktionsgemisch auf eine Mischung aus 100 ml Eiswasser und 50 ml Eisessig gegossen, und der erhaltene Niederschlag wurde mit 10 Gew.-%iger Natriumhydrogencarbonatlösung gewaschen. Ausbeute: 81 %, Fp.: 107-109°C.

Beispiel 2

N-(Cyclohexylidenimino-oxycarbonylmethyl)-1,8-naphthalindicarbonsäureimid

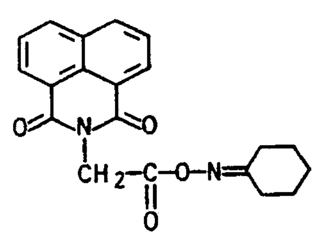

Das in Stufe 1.1 erhaltene Carbonsäurechlorid wurde analog Beispiel 1.2 mit 6,2 g (55 mmol) Cyclohexanonoxim umgesetzt. Ausbeute: 71 %; Fp.: 184-185°C.

Beispiel 3

N-(1-Methyl-1-ethoxymethylenimino-oxycarbonylmethyl)-1,8-naphthalindicarbonsäureimid

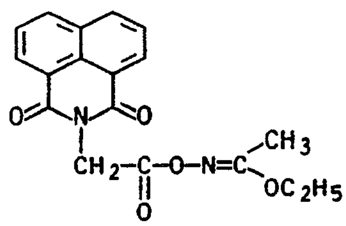

Das in Stufe 1.1 erhaltene Carbonsäurechlorid wurde analog Beispiel 1.2 mit 5,9 g (55 mmol) Acethydroxamsäureethylether umgesetzt. Nach der Hydrolyse mit Eiswasser/Eisessig wurde die gesamte Mischung bis zur Trockene eingeengt. Danach wurde der Rückstand in wässriger Natriumhydrogencarbonatlösung aufgenommen und dreimal mit je 120 ml Essigester extrahiert. Nach Einengen der vereinigten Extrakte wurde der verbleibende Feststoff aus Ethanol umkristallisiert. Ausbeute: 10 % (E/Z-Isomerengemisch); Fp.: 120 bis 125° C.

Beispiel 4

N-(Benzylidenimino-oxycarbonylmethyl)-1,8-naphthalindicarbonsäureimid

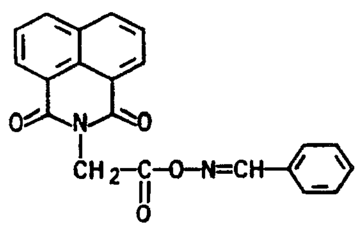

Das in Stufe 1.1 erhaltene Carbonsäurechlorid wurde analog Beispiel 1.2 zu 6,7 g (55 mmol) Benzaldoxim in 50 ml Pyridin gegeben. Anschließend wurde 60 h bei Raumtemperatur gerührt, wie in Beispiel 1.2 beschrieben aufgearbeitet, und das Produkt wurde aus Pyridin umkristallisiert. Ausbeute: 53 %; Fp.: 195-196° C.

Beispiel 5

N-(4-Chlorbenzylidenimino-oxycarbonylmethyl)-1,8-naphthalindicarbonsäureimid

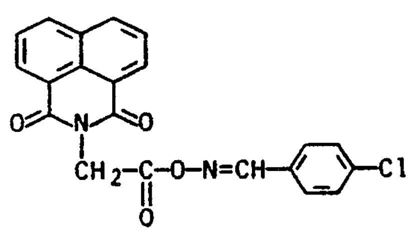

Das in Stufe 1.1 erhaltene Carbonsäurechlorid wurde analog Beispiel 1.2 mit 8,6 g (55 mmol) 4-Chlorbenzaldoxim umgesetzt, und das Produkt wurde anschließend aus Pyridin umkristallisiert. Ausbeute: 52 %; Fp.: 204° C.

Beispiel 6 (Verbindung Nr. 101):

N,N'-Bis(hydroxycarbonylmethyl)-1,4,5,8-naphthalintetracarbonsäurediimid

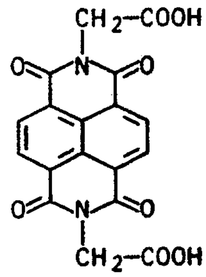

Eine Mischung aus 26,8 g (0,1 mol) 1,4,5,8-Naphthalintetracarbonsäuredianhydrid, 15,0 g (0,2 mol) Glycin und 200 ml Dimethylformamid wurde 10 Stunden lang auf 140 °C erhitzt und nach dem Abkühlen in 1 1 Wasser eingegossen, um das Produkt auszufällen. Der erhaltene Niederschlag wurde abgetrennt und getrocknet. Ausbeute: 75%; Fp.: > 300 °C.

Die in Tabelle 1 aufgeführten Verbindungen der Formel Ia wurden analog den Beispielen 1 - 5 synthetisiert oder können entsprechend hergestelt werden. In den Tabellen 2 und 3 sind Vertreter der Verbindungen Ib bzw. Ic aufgeführt.

Synthesebeispiele für Cyclohexenone der Formel V, in der $R^e$ für einen unsubstituierten oder substituierten Butenylphenylrest steht:

Herstellvorschrift für Beispiel V.45 aus Tabelle B

a) Zu einer Lösung von 93,1 g (1 mol) Anilin in 340 ml Wasser und 225 ml konz. Salzsäure gab man bei 0 °C 69,1 g (1 mol) Natriumnitrit in 100 ml Wasser.

b) In 840 ml Aceton und 50 ml Wasser wurden bei -15 °C 67,6 g (1,25 mol) Butadien eingegast, 15,5 g Kupfer(II)chlorid und 22,5 g Calziumoxid zugegeben und anschließend innerhalb 2 Stunden mit der unter a) hergestellten Diazoniumsalzlösung versetzt. Diese Reaktionsmischung ließ man auf 25 °C erwärmen. Nach 6 Stunden Rühren wurde mit Methyl-t-butylether extrahiert, das organische Extrakt eingeengt und über einen Dünnfilmverdampfer (0,2 Torr; 80 °C) destilliert. Man erhielt so in 55 % Gesamtausbeute ein Gemisch aus 1-chlor-4-phenylbut-2-en und 3-Chlor-4-phenylbut-1-en (78:22).

c) In 480 ml trockenes N-Methylpyrrolidon gibt man nacheinander 78,3 g (0,48 mol) N-Hydroxyphthalimid und 44,2 g (0,32 mol) Kaliumcarbonat. Bei 40 °C Innentemperatur tropft man 88,8 g (0,54 mol) der nach Beispiel b) erhaltenen Chloridmischung zu. Man erwärmt auf 60 °C und rührt weitere 6 Stunden. Nach Abkühlen gießt man auf 2 Liter Eiswasser und filtriert ab. Nach Waschen und Trocknen erhält man 90 % d.Th. an (E)-N-(4-Phenyl-2-butenyloxy)-phthalimid. Fp.: 70-71 °C (Isopropanol)

d) 55,5 g (0,19 mol) des Phthalimidethers e) in 190 ml Essigsäureethylester wurden bei 60 °C unter Rühren mit 11,6 g (0,19 mol) Ethanolamin versetzt. Nach 5 Stunden wurde das ausgefallene N-(Hydroxyethyl)-phthalimid abfiltriert und das Filtrat mit 18,8 g Oxalsäure in 30 ml Essigsäureethylester versetzt. Man erhielt so 95 % d.Th. an (E)-4-Phenyl-2-butenyloxy-amin als Oxalat-Salz. Fp.: 127-129 °C.

e) 4,3 g (0,016 mol) 2-Propionyl-5-(3-tetrahydrothiopyranyl)-cyclohexan-1,3-dion, 4,5 g (0,018 mol) 4-Phenyl-but-2-enyloxiammoniumoxalat und 3,0 g Natriumhydrogencarbonat wurden in 100 ml Methanol 16 Stunden bei 25 °C gerührt. Das Lösungsmittel wurde bei vermindertem Druck abdestilliert und der Rückstand an Kieselgel mit einem Toluol/Essigsäureethylester-Gemisch im Volumenverhältnis 8:2 chromatographiert.

Nach dem Entfernen des Lösungsmittels erhielt man 2,2 g (34,3 % d.Th.) 3-Hydroxy-2-[1-(4-Phenyl-but-2-enyloximino)-propyl]-5-(3-tetrahydrothiopyranyl)-cyclohex-2-en-1-on als Harz.

Soweit in der Tabelle B nichts anderes angegeben ist, liegen die Alkenylreste in der E-Konfiguration vor. Die $^1$H-NMR-Angaben beziehen sich auf charakteristische angewählte Signale.

Synthesebeispiele für Cyclohexenone der Formel V, in der $R^e$ für einen unsubstituierten oder substituierten Phenylbutylrest steht:

Herstellvorschrift für Beispiel V.56 aus Tabelle C

a) N(4-(4-Fluorphenyl)butyloxy)phthalimid

71,5 g (0,23 mol) N-(4-(4-Fluorphenyl)-3-butenyloxy)phthalimid (hergestellt nach DE-OS 38 38 310) löst man in 300 ml Tetrahydrofuran, fügt 2 g Palladium auf Aktivkohle (10 %ig) zu und hydriert bei leichtem Überdruck bis das I,2-fache der theoretischen Wasserstoffmenge verbraucht worden ist.

Man saugt über Kieselgur ab, engt ein und kristallisiert aus Isopropanol um.

Ausbeute: 62,8 g (87 %); Fp.: 67-68 °C

250-MHz-$^1$H-NMR (DMSO-$d_6$)

$\delta$ (ppm) = 1,8-1,9 (m, 4H); 2,65 (t,2H); 4,15 (t,2H) ; 7,0-7,35 (m, 4H);
7,86 (s,4H)

b) 61,8 g (0,197 mol) des zuvor hergestellten Phthalimidethers trägt man portionsweise in 92 ml Ethanolamin ein. Nachdem man 3 h auf 60° C erhitzt hat gießt man nach Erkalten in 400 ml Eiswasser und extrahiert dreimal mit 100 ml Dichlormethan. Die vereinten organischen Phasen werden mit gesättigter Kochsalzlösung gewaschen, getrocknet und im Vakuum eingeengt.
Man isoliert so das 4-(4-Fluorphenyl)butyloxyamin als Öl.
250-MHz-$^{1}$H-NMR ($CDCl_3$)
$\delta$ (ppm) = 1,5-1,75 (m, 4H); 2,61 (t,2H) 3,68 (t,2H);
5,4 (breites s, 2H); 6,9-7,2 (m,4H)

c) 2-{1-[4-(4-Fluorphenyl)-butyloximino]-propyl}-5-tetrahydrothiopyran-3-yl-3-hydroxy-cyclohex-2-enon

3 g (1 mmol) 2-Butyryl-3-hydroxy-5-tetrahydrothiopyran-3-yl-cyclohex-2-enon wird in 100 ml trockenem Methanol gelöst und mit 2,2 g (12 mmol) 4-(4-Fluorphenyl)butyloxyamin versetzt. Das Gemisch wird 16 h bei Raumtemperatur gerührt und wird dann im Vakuum zur Trockene eingedampft. Der Rückstand wird in Diethylether aufgenommen und an Kieselgel chromatographiert. Man erhält 3,6 g (80 % d. Th.) der Titelverbindung.

Tabelle 1

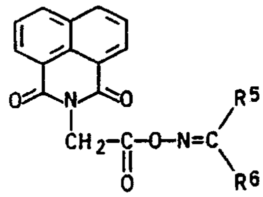

Ia ($R^1$-$R^4$=H; Y,Z=O)

| Bsp.-Nr. | $R^5$ | $R^6$ | Fp. [°C] |
|---|---|---|---|
| 1 | $CH_3$ | $CH_3$ | 107-109 |
| 2 | $-CH_2-(CH_2-)_3-CH_2-$ | | 184-185 |
| 3 | $CH_3$ | $OC_2H_5$ | 120-125 |
| 4 | H | Phenyl | 195-196 |
| 5 | 4-Cl-Phenyl | H | 204 |
| 6 | $CO_2CH_3$ | $CH_3$ | 110-114 |
| 7 | 4-Cl-Phenyl | $CH_3$ | 182-190 |
| 8 | Phenyl | CN | 200-201 |
| 9 | $C_2H_5$ | $CH_3$ | |
| 10 | $CH_3$ | $i-C_3H_7$ | |
| 11 | $n-C_3H_7$ | $n-C_3H_7$ | |
| 12 | $i-C_3H_7$ | $i-C_3H_7$ | |
| 13 | $i-C_4H_9$ | $i-C_4H_9$ | |
| 14 | $CH_3$ | Neopentyl | |
| 15 | $CH_3$ | $-(CH_2)_3-CH(CH_3)_2$ | |
| 16 | $CH_3$ | $-(CH_2)_2-CH=C(CH_3)_2$ | |
| 17 | $CH_3$ | $CH_2-OCH_3$ | |
| 18 | $C_2H_5$ | $CH_2-OCH_3$ | |
| 19 | 4-Cl-Phenyl | $CH_3$ | |
| 20 | $-CH(CH_3)-S-CH_3$ | $CH_3$ | |
| 21 | Benzyl | $CH_3$ | |
| 22 | Phenyl | $CH_3$ | |
| 23 | Phenyl | $CH_2Cl$ | |
| 24 | Phenyl | 1,2,4-Triazolylmethyl | |
| 25 | $CO-CH_3$ | $CH_3$ | |
| 26 | $CO-CH_3$ | Phenyl | |
| 27 | $4-N(CH_3)_2$-Phenyl | H | |
| 28 | 4-Cl-Phenyl | H | |
| 29 | 3-Cl-Phenyl | H | |
| 30 | 4-F-Phenyl | H | |
| 31 | $4-CF_3$-Phenyl | H | |
| 32 | $4-CH_3$-Phenyl | H | |
| 33 | $4-(CH_3-O)$-Phenyl | H | |
| 34 | $3-CH_3$-Phenyl | H | |
| 35 | $3-(CH_3-O)$-Phenyl | H | |

Tabelle 1 (Fortsetzung)

| Bsp.-Nr. | $R^5$ | $R^6$ | Fp. [°C] |
|---|---|---|---|
| 36 | 3-($CHF_2$-$CF_2$-O)-Phenyl | H | |
| 37 | 4-($CH_3$-COHN)-Phenyl | H | |
| 38 | 4-CN-Phenyl | H | |
| 39 | 3,4-$Cl_2$-Phenyl | H | |
| 40 | 2,4-$Cl_2$-Phenyl | H | |
| 41 | 2,4-(n-$C_6H_{13}$-S)-Phenyl | H | |
| 42 | $CH_3$ | 2-Thienyl | |
| 43 | $CH_3$ | 3-Thienyl | |
| 44 | H | 2-Furyl | |
| 45 | $CH_3$ | 2-Furyl | |
| 46 | H | 5,6-Dihydro-$\Delta^3$-thiopyran-3-yl | |
| 47 | $CH_3$ | 5,6-Dihydro-$\Delta^3$-pyran-3-yl | |
| 48 | 2-Furyl | n-$C_3H_7$ | |
| 49 | $CH_3$ | 2,3-Dihydro-6-$CH_3$-$\Delta^5$-pyran-6-yl | |
| 50 | 4-(OH)-3,5-$Br_2$-Phenyl | H | |
| 51 | 4-(OH)-3,5-$I_2$-Phenyl | H | |
| 52 | CO-O-$C_2H_5$ | CN | |
| 53 | $-CH_2-(CH_2)_2-CH_2-$ | | |
| 54 | $-CH_2-C(CH_3)_2-CH_2-CH(CH_3)-$ | | |
| 55 | $-CH=C(CH_3)-CH_2-C(CH_3)_2-CH_2-$ | | |
| 56 | $-CH_2-CH(CH_3)-CH_2-CH_2-$ | | |
| 57 | $-CH_2-CH(CH_3)-(CH_2)_2-CH_2-$ | | |
| 58 | $-CH=C(CH_3)-CH_2-CH_2-$ | | |
| 59 | $-CH=CH-(CH_2)_2-CH_2-$ | | |
| 60 | $-CH_2-(CH_2)_4-CH_2-$ | | |
| 61 | $-CH=C(CH_3)-O-C(CH_3)=CH-$ | | |
| 62 | $-CH=C(CH_3)-S-C(CH_3)=CH-$ | | |
| 63 | $-CH_2-CH_2-S-CH_2-CH_2-$ | | |
| 64 | $-CH_2-CH_2-O-CH_2-CH_2-$ | | |
| 65 | $-CH_2-(CH_2)_9-CH_2-$ | | |
| 66 | 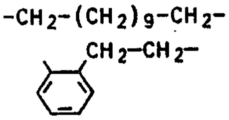 | | |
| 67 | 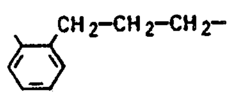 | | |
| 68 | $-CH_2-(CH_2)_2-CH=C(OC_2H_5)-$ | | 178-182 |

Tabelle 2

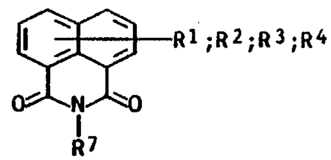

Ib (Y,Z=O)

| Bsp.-Nr. | $R^1$ | $R^2$ | $R^3$ | $R^4$ | $R^7$ | Fp. [°C] | Literatur *) |
|---|---|---|---|---|---|---|---|
| 68 | H | H | H | H | $CH_2COOH$ | 270-272 | [1] |
| 69 | H | H | H | H | H | > 300 | [2] |
| 70 | H | H | H | H | $CH_2COOCH_3$ | 175-178 | |
| 71 | H | H | H | H | $CH_2COO-i-C_3H_7$ | 126 | |
| 72 | H | H | H | H | $CH_3$ | 210-213 | [3] |
| 73 | H | H | H | H | $CH(CH_3)COOH$ | 251 | [1] |
| 74 | H | H | H | H | $CH_2CH_2COOH$ | 240-243 | [1] |
| 75 | H | H | H | H | 4-(COOH)-Phenyl | > 300 | |
| 76 | H | 4-Cl | H | H | $CH_2COOH$ | 265 | [4] |
| 77 | H | $3-NO_2$ | H | H | $CH_2COOH$ | 220-222 | [5] |
| 78 | H | H | H | H | $n-C_3H_6-COOH$ | 180-182 | [6] |
| 79 | H | H | H | H | $CH_2CONH-OCH_3$ | 265-269 | |
| 80 | H | H | H | H | $CH_2COO^{\ominus}Na^{\oplus}$ | > 300 | [7] |
| 81 | H | H | H | H | $CH_2CON(CH_3)-OCH_3$ | | |
| 82 | H | H | H | H | $CH_2COO$-(2,5-dioxo-pyrrolidin-1-yl) | 270-275 | |
| 83 | H | H | H | H | $CH_2SO_3H$ | | |

Tabelle 2 (Fortsetzung)

| Bsp.-Nr. | $R^1$ | $R^2$ | $R^3$ | $R^4$ | $R^7$ | Fp. [°C] | Literatur *) |
|---|---|---|---|---|---|---|---|
| 84 | H | H | H | H | $CH_2COO^{\ominus\oplus}NH(n\text{-}C_4H_9)_3$ | 172 | |
| 85 | H | H | H | H | $CH_2CH_2N(CH_3)_2$ | | |
| 86 | H | $4\text{-}NH_2$ | H | H | H | | |
| 87 | H | $4\text{-}SCH_3$ | H | H | $n\text{-}C_4H_9$ | | |
| 88 | H | $4\text{-}SCH_3$ | H | H | $n\text{-}C_6H_{13}$ | | |
| 89 | H | $4\text{-}SO_2CH_3$ | H | H | $n\text{-}C_4H_9$ | | |
| 90 | 3-Br | $4\text{-}NH\text{-}CH_2\text{-}CH(C_2H_5)\text{-}C_4H_9$ | H | H | $CH_3$ | | |
| 91 | H | $4\text{-}SCH_2\text{-}COOH$ | H | H | $n\text{-}C_4H_9$ | 145-148 | |
| 92 | H | H | H | H | $CH_2COO^{\ominus}$ 1/2 $Ca^{2+}$ | > 300 | |
| 93 | 4-Cl | H | H | H | OH | 219-226 | |
| 94 | 4-Cl | H | H | H | $O\text{-}CH_2CH_3$ | 170-175 | |
| 95 | H | $4\text{-}SCH_2CO_2H$ | H | H | $O\text{-}CH_2CH_3$ | 212-220 | |
| 96 | H | $4\text{-}SCH_3$ | H | H | $O\text{-}CH_2CH_3$ | 177-182 | |
| 97 | 4-Cl | H | H | H | $n\text{-}C_4H_9$ | | |

*) Die Herstellung dieser Verbindungen ist in der genannten Literatur sowie in den dort aufgeführten Zitaten beschrieben oder erfolgt nach analogen Methoden.

Literaturverzeichnis:


[1] A.M. El-Naggar et al., Egypt. J. Chem. 24, 127 (1981);

[2] K.K. Hatzios & P. Zama, Pestic. Sci. 17, 25 (1986);

[3] P.H. Mazzocchi et al., Tetrahedron Lett. 29, 513 (1988);

[4] US-A-4,254,108;

[5] J.J. Ares et al., J. Med. Chem. 29, 2384 (1986);

[6] FR-A-2 521 139;

[7] A.S. Efimov et al., Probl. Endokrinol. 26, 69 (1980).

Tabelle 3

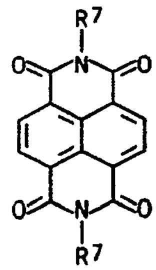

Ic ($R^1$; $R^2$ = H)

| Bsp.-Nr. | $R^7$ | Fp. [°C] | Herst. *) |
|---|---|---|---|
| 101 | $CH_2-COOH$ | > 300 | |
| 102 | $CH_2-COO^{\ominus}$ $H_3N^{\oplus}(i-C_3H_7)$ | > 300 | |
| 103 | $CH_2-COO^{\ominus}$ $Na^{\oplus}$ | > 300 | |
| 104 | $CH_2-CO-OCH_3$ | > 250 | |
| 105 | $CH_2-CO-OCH(CH_3)_2$ | > 250 | |

*) Die Herstellung dieser Verbindungen ist in Beispiel 6 beschrieben oder erfolgt nach analogen Methoden.

Beispiele zur biologischen Wirkung

Der Einfluß verschiedener Vertreter der erfindungsgemäßen herbiziden Mittel bzw. Mittelkombinationen, bestehend aus Herbizid und antidotisch wirkender Verbindung, auf das Wachstum von erwünschten und unerwünschten Pflanzen im Vergleich zum herbiziden Wirkstoff allein wird durch die folgenden biologischen Beispiele aus Gewächshausversuchen belegt:

Bei Gewächshausversuchen dienten als Kulturgefäße Plastikblumentöpfe mit rund 300 $cm^3$ Inhalt und lehmigem Sand mit etwa 3,0 % Humus als Substrat. Die Samen der Testpflanzen wurden nach Arten getrennt, flach eingesät und befeuchtet. Danach wurden die Gefäße mit durchsichtigen Plastikhauben abgedeckt, bis die Samen gleichmäßig gekeimt und die Pflanzen angewachsen waren.

Für die Nachauflaufbehandlung zieht man die Testpflanzen je nach Wuchsform erst bis zu einer Wuchshöhe von 3 bis 20 cm angezüchtet und erst dann behandelt. Die herbiziden Mittel wurden hierbei in Wasser als Verteilungsmittel suspendiert oder emulgiert und mittels fein verteilender Düsen gespritzt.

Als Beispielsherbizid der Cyclohexenonderivate der Formel V dienten

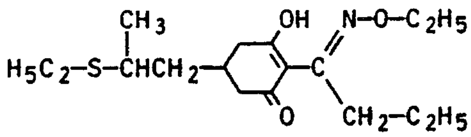

V.2,

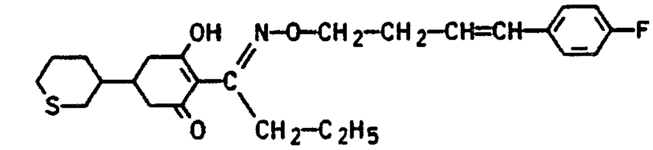

V.44,

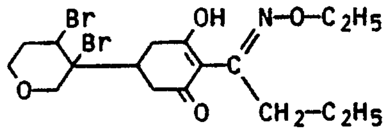

V.20.

Der herbizide Wirkstoff V.2 wurde als kommerziell formuliertes Produkt (184 g/l Emulsionskonzentrat) in die Aufbereitung der jeweiligen antidotische wirkenden Verbindung gegeben und so gemeinsam mit dieser ausgebracht.

Für einen Vergleichsversuch wurde der kommerziell formulierte Wirkstoff V.2 mit einer Mischung aus 80 % Cyclohexenon und 20 % Emulphor EL *) (Leerformulierung, ohne Antidot) appliziert.

Der herbizide Wirkstoff V.44 wurde als 10 Gew.-%ige Lösung auf die Pflanzen appliziert. Die Herstellung der Lösung erfolgte durch einmischen des Wirkstoffs in eine Lösung aus 93 % Xylol und 7 % Lutensol®AP-8 **) .

Das Beispielherbizid V.20 wurde als Lösung appliziert, die 200 g/l in Solvesso 200 ***) enthielt.

Sämtliche antidotisch wirkende Verbindungen wurden für die Nachauflaufbehandlung in einem Gemisch, bestehend aus 80 % Cyclohexenon und 20 % Emulphor EL mit 10 % (Gew.-%) Wirkstoff aufbereitet.

Liste der Testpflanzen

| Lateinischer Name | Deutscher Name | Englischer Name |
|---|---|---|
| Alopecurus myosuroides | Ackerfuchsschwanz | blackgrass |
| Lolium multiflorum | Ital. Raygrass | annual ryegras |
| Triticum aestivum | Weizen | wheat |
| Setaria italica | Kolbenhirse | foxtail millet |
| Zea mays | Mais | Indian corn |

Die Versuchsgefäße wurden im Gewächshaus aufgestellt, wobei für wärmeliebende Arten 18 bis 30 °C und für solche gemäßigterer Klimate (10 bis 25 °C) bevorzugt wurden.

Zu Freilandversuchen wurde ein Versuchsfeld in Kleinparzellen von 2 $m^2$ unterteilt und die Testpflanzen in Reihen eingesäht. Der Boden des Feldes bestand aus lehmigem Sand mit ca. 1,5 % Humus, er hatte

*) ethoxyliertes Rizinusöl (castor-oil)

**) nichtionisches oberflächenaktives Mittel auf Basis von Alkylphenolpolyethylenglykolethern

***) Solvesso® = mehr als 97 Vol-% Aromaten enthaltendes Lsgm. von bes. Reinheitsgrad für Farben, Lacke, Schädlingsbekämpfungsmittel

einen pH-Wert von 6. Die Mittel wurden in Wasser als Träger und Verteilungsmittel suspendiert und mit Hilfe einer mobilen Spritzkabine, die fein verteilende Düsen hatte, im Nachauflaufverfahren auf die Pflanzen tropfnaß aufgebracht.

Die Versuchsperiode erstreckte sich über 3 bis 4 Wochen. Während dieser Zeit wurden die Pflanzen gepflegt, und ihre Reaktion auf die einzelnen Behandlungen wurde erfaßt.

Bewertet wurde die Schädigung durch die chemischen Mittel anhand einer Skala von 0 bis 100 % im Vergleich zu den unbehandelten Kontrollpflanzen. Dabei bedeutet 0 keine Schädigung und 100 eine völlige Zerstörung der Pflanzen.

Die anschließenden Tabellen dokumentieren die antidotische Wirkung der erfindungsgemäßen Beispielsverbindungen Nr. 68, 86, 87, 88 und 89.

Dabei verbessern diese Beispielsverbindungen deutlich die Verträglichkeit des Herbizids V.2 für Kulturpflanzen. Im Freiland verbessert die Beispielsverbindung Nr. 68 deutlich die Verträglichkeit des Herbizids V.6 für Mais, besonders aber die Verträglichkeit des Herbizids V.20 für Weizen.

Tabelle 1: Verbesserung der Verträglichkeit des Herbizids V.2 für Weizen durch Zumischen einer antidotischen Beispielsverbindung und Nachauflaufanwendung; Gewächshausversuch

| Herbizid Nr. | Antidot Nr. | Aufwandmenge kg/ha a.S. | Testpflanzen und Schädigung % Kulturpflanze Triticum aestivum* | unerwünschte Lolium multif. | Pflanze Setaria italica |
|---|---|---|---|---|---|
| V.2 | - | 0,03 | 70 | 98 | - |
| V.2 | 68 | 0,03+0,125 | 10 | 95 | - |
| V.2 | 87 | 0,03+0,125 | 10 | 95 | - |
| V.2 | 89 | 0,03+0,125 | 10 | 95 | - |
| V.2 | - | 0,015 | 40 | - | 100 |
| V.2 | 86 | 0,015+0,06 | 20 | - | 100 |

*Sorte: "Okapi"

Tabelle 2: Verbesserung der Verträglichkeit des Herbizids Nr. V.20 für Weizen in Nachauflaufanwendung durch Zumischen einer antidotischen Beispielsverbindung; Freilandversuch

| | | | Testpflanzen und Schädigung % | |
|---|---|---|---|---|
| Herbizid Nr. | Antidot Nr. | Aufwandmenge kg/ha a.S. | Kulturpflanze Triticum aestivum* | unerwünschte Pflanze Alopecurus myosuroides |
| V.20 | - | 0,5 | 35 | 95 |
| V.20 | 68 | 0,5+1,0 | 2 | 90 |

*Sorte: "Okapi"

Tabelle 3: Verbesserung der Verträglichkeit des Herbizids Nr. V.2 für Mais in Nachlaufanwendung durch Zumischen einer antidotischen Beispielsverbindung; Gewächshausversuch

| | | | Testpflanzen und Schädigung % | |
|---|---|---|---|---|
| Herbizid Nr. | Antidot Nr. | Aufwandmenge kg/ha a.S. | Kulturpflanze Zea mays* | unerwünschte Pflanze Setaria italia |
| V.2 | - | 0,015 | 85 | 95 |
| V.2 | 88 | 0,015+0,06 | 20 | 95 |

*Sorte: "Mutin"

Tabelle 4: Verbesserung der Verträglichkeit des Herbizids Nr. V.6 für Mais in Nachauflaufanwendung durch Zumischen einer antidotischen Beispielsverbindung; Freilandversuch

| | | | Testpflanzen und Schädigung % | |
|---|---|---|---|---|
| Herbizid Nr. | Antidot Nr. | Aufwandmenge kg/ha a.S. | Kulturpflanze Zea mays* | unerwünschte Pflanze Setaria italia |
| V.44** | - | 0,5 | 58 | 100 |
| V.44** | 68 | 0,5+0,25 | 15 | 100 |

* Sorte "Mutin"
**mit der Wirkstoffaufbereitung wurde noch 1 l/ha Netzmittel ausgebracht.

## Ansprüche

1. Substituierte 1,8-Naphthalindicarbonsäureimide der allgemeinen Formel Ia

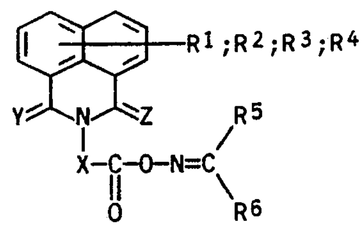

Ia

in der die Substituenten die folgende Bedeutung haben:

$R^1$ - $R^4$ Wasserstoff, Halogen, Hydroxy, Mercapto, Cyano, Thiocyanato, Nitro, $C_1$-$C_6$-Alkylgruppen, die unsubstituiert oder partiell oder vollständig halogeniert sein können, $C_2$-$C_6$-Alkenylgruppen, $C_2$-$C_6$-Alkinylgruppen, $C_3$-$C_8$-Cycloalkyl-oder $C_3$-$C_8$-Cycloalkoxygruppen, die Aminogruppe, $C_1$-$C_8$-Alkylamino- oder Di-($C_1$-$C_6$)-alkylaminogruppen, Amino-$C_1$-$C_4$-alkylgruppen, $C_1$-$C_6$-Alkylamino-$C_1$-$C_4$-alkyl-oder Di-($C_1$-$C_6$)-alkylamino-$C_1$-$C_4$-alkylgruppen, $C_1$-$C_4$-Alkoxygruppen, $C_1$-$C_4$-Alkylthiogruppen, $C_1$-$C_4$-Alkoxy-$C_1$-$C_4$-alkylgruppen, $C_1$-$C_4$-Alkylthio-$C_1$-$C_4$-alkylgruppen, $C_1$-$C_6$-Alkylcarbonyl- oder $C_1$-$C_6$-Alkylcarbonyloxygruppen, $C_1$-$C_6$-Alkoxycarbonylgruppen, die Hydroxycarbonylgruppe, Hydroxycarbonyl-$C_1$-$C_4$-alkylgruppen, Hydroxycarbonyl-$C_1$-$C_4$-alkoxy- oder Hydroxycarbonyl-$C_1$-$C_4$-alkylthiogruppen, die Hydroxysulfonylgruppe, $C_1$-$C_6$-Alkylsulfinyl- oder $C_1$-$C_6$-Alkylsulfonylgruppen, $C_1$-$C_4$-Alkoxysulfonylgruppen, die Aminosulfonylgruppe, $C_1$-$C_4$-Alkylaminosulfonyl- oder Di-($C_1$-$C_4$)-alkylaminosulfonylgruppen, die Hydrazinogruppe, die bis zu drei $C_1$-$C_4$-Alkylreste tragen kann, die Phenylgruppe, Phenyl-$C_1$-$C_3$-alkylgruppen, die Naphthylgruppe, Naphthyl-$C_1$-$C_3$-alkylgruppen, 5- oder 6-gliedrige Heteroaryl- oder Heteroaryl-$C_1$-$C_3$-alkylgruppen, die Phenoxy- oder Naphthoxygruppe, die Phenylthio- oder Naphthylthiogruppe, die Phenoxycarbonyl-oder Naphthoxycarbonylgruppe, die Phenylcarbonyl-oder Naphthylcarbonylgruppe, die Phenylcarbonyloxy-oder Naphthylcarbonyloxygruppe, Phenyl-$C_1$-$C_3$-alkoxy-oder Naphthyl-$C_1$-$C_3$-alkoxygruppen, 5- oder 6-gliedrige Heteroaryl-$C_1$-$C_3$-alkoxygruppen, die Phenylsulfinyl- oder Naphthylsulfinylgruppe, 5- oder 6-gliedrige Heteroarylsulfinylgruppen, die Phenylsulfonyl-oder Naphthylsulfonylgruppe, 5- oder 6-gliedrige Heteroarylsulfonylgruppen, die Phenoxysulfonyl- oder Naphthoxysulfonylgruppe, 5- oder 6-gliedrige Heteroaryloxysulfonylgruppen, wobei die Aryl- und Heteroarylteile der genannten Substituenten noch bis zu drei der folgenden Substituenten tragen können: Halogen, Cyano, Nitro, $C_1$-$C_4$-Alkyl, $C_1$-$C_4$-Alkoxy, Amino, $C_1$-$C_6$-Alkylamino, Di-($C_1$-$C_6$)-alkylamino oder partiell oder vollständig halogeniertes $C_1$-$C_4$-Alkyl;

x eine $C_1$-$C_6$-Alkylenkette, die noch einen der folgenden Reste tragen kann: Halogen, Hydroxy, Mercapto, $C_1$-$C_4$-Alkyl, $C_1$-$C_4$-Alkoxy, $C_1$-$C_4$-Alkylthio, Di-($C_1$-$C_4$)-alkylamino, $C_1$-$C_4$-Alkoxycarbonyl, Cyano, $C_1$-$C_6$-Alkylcarbonyl, $C_1$-$C_6$-Alkylcarbonyloxy, Phenyl, Naphthyl, 5- oder 6-gliedriges Heteroaryl, Phenyl-($C_1$-$C_3$)-alkyl, Naphthyl-($C_1$-$C_3$)-alkyl oder 5- oder 6-gliedriges Heteroaryl-($C_1$-$C_3$)-alkyl, wobei die Aryl- und Heteroarylteile der letztgenannten sechs Reste noch bis zu 3 Halogenatome oder $C_1$-$C_4$-Alkylgruppen tragen können;

$R^5$ Wasserstoff, Cyano, eine $C_1$-$C_6$-Alkylgruppe, eine $C_2$-$C_6$-Alkenylgruppe, eine $C_1$-$C_4$-Alkoxy-$C_1$-$C_4$-alkylgruppe, eine $C_1$-$C_4$-Alkylthio-$C_1$-$C_4$-alkylgruppe, eine $C_1$-$C_4$-Alkoxycarbonylgruppe, die Acetylgruppe, eine $C_3$-$C_6$-Cycloalkylgruppe, die Benzoyl-, Naphthoyl- oder eine 5- oder 6-gliedrige Heteroaroylgruppe, die Phenyl- oder Naphthylgruppe, eine Phenyl-$C_1$-$C_3$-alkyl- oder Naphthyl-$C_1$-$C_3$-alkylgruppe, eine 5- oder 6-gliedrige Heteroaryl- oder Heteroaryl-$C_1$-$C_3$-alkylgruppe, wobei die Aryl- und Heteroarylteile der letztgenannten sechs Gruppen noch bis zu drei der folgenden Reste tragen können: Halogen, Hydroxy, Cyano, Trifluormethyl, $C_1$-$C_6$-Alkoxy, das unsubstituiert oder partiell oder vollständig halogeniert sein kann, $C_1$-$C_6$-Alkylthio, $C_1$-$C_6$-Alkyl, Di-($C_1$-$C_3$)-alkylamino oder Acetamino;

$R^6$ Wasserstoff, Cyano, eine $C_1$-$C_6$-Alkylgruppe, die unsubstituiert oder partiell oder vollständig halogeniert sein kann, eine $C_1$-$C_4$-Alkoxygruppe, eine $C_1$-$C_4$-Alkoxy-$C_1$-$C_4$-alkylgruppe, eine Diazolylmethyl- oder Triazolylmethylgruppe, eine $C_1$-$C_6$-Alkoxycarbonylgruppe, die Furyl- oder Tetrahydrofurylgruppe, die Thienylgruppe, eine Dihydropyranyl- oder Dihydrothiopyranylgruppe, die Tetrahydropyranyl-oder Tetrahydrothiopyranylgruppe, oder
eine Phenylgruppe, wenn $R^5$ Wasserstoff, Methyl oder Acetyl bedeutet;
oder
zusammen mit dem Rest $R^5$ und dem gemeinsamen C-Atom einen $C_3$-$C_{12}$-Cycloalkylrest, einen 4-Oxocyclohexadienylrest oder einen 5- oder 6-gliedrigen Ring, der gesättigt oder partiell oder vollständig ungesättigt sein kann und ein Sauerstoff- oder Schwefelatom als Heteroatom enthalten kann und/oder der noch bis zu drei $C_1$-$C_3$-Alkyl- oder $C_1$-$C_3$-Alkoxygruppen oder einen anellierten Benzolring tragen kann;

Y, Z Sauerstoff oder Schwefel;

mit der Maßgabe, daß $R^5$ und $R^6$ nicht gleichzeitig Wasserstoff bedeuten, sowie die pflanzenverträglichen Salze derjenigen Verbindungen Ia, bei denen $R^1$; $R^2$; $R^3$; $R^4$ Hydroxycarbonyl, Hydroxycarbonyl-$C_1$-$C_4$-alkyl, Hydroxycarbonyl-$C_1$-$C_4$-alkoxy, Hydroxycarbonyl-$C_1$-$C_4$-alkylthio oder Hydroxysulfonyl bedeuten.

2. Verfahren zur Herstellung der Verbindungen Ia gemäß Anspruch 1, dadurch gekennzeichnet, daß man ein Säurehalogenid der allgemeinen Formel II

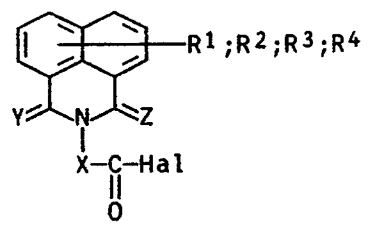

II,

wobei Hal für Fluor, Chlor oder Brom steht, mit einem Oxim der allgemeinen Formel III

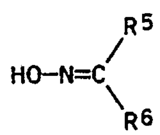

III

umsetzt.

3. Herbizide Mittel, enthaltend mindestens ein substituiertes 1,8-Naphthalindicarbonsäureimid der allgemeinen Formel Ia gemäß Anspruch 1 und/oder ein 1,8-Naphthalindicarbonsäureimid der allgemeinen Formel Ib

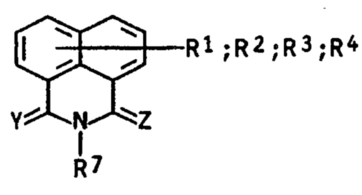

Ib

in der $R^7$ die folgende Bedeutung hat:

a) Wasserstoff, Hydroxy, Cyano, eine $C_1$-$C_6$-Alkylgruppe, die unsubstituiert oder partiell oder vollständig halogeniert sein kann, eine $C_1$-$C_6$-Hydroxyalkylgruppe, eine $C_2$-$C_6$-Alkenylgruppe, eine $C_1$-$C_4$-Alkoxygruppe, eine $C_1$-$C_4$-Alkoxy-$C_1$-$C_4$-alkylgrupe, eine $C_1$-$C_4$-Alkylthio-$C_1$-$C_4$-alkylgruppe, die Aminogruppe, eine $C_1$-$C_4$-Alkylaminogruppe, eine Di-($C_1$-$C_4$)-alkylaminogruppe, eine $C_3$-$C_8$-Cycloalkylgruppe, eine $C_3$-$C_8$-Cycloalkoxygruppe, eine Acylgruppe, eine $C_1$-$C_4$-Alkoxycarbonyl-$C_1$-$C_6$-alkylgruppe, eine Aminocarbonyl-$C_1$-$C_6$-alkylgruppe, eine $C_1$-$C_4$-Alkylaminocarbonyl-$C_1$-$C_6$-alkylgruppe, eine Di($C_1$-$C_4$)-alkylaminocarbonyl-$C_1$-$C_6$-alkylgruppe, eine $C_1$-$C_4$-Alkoxyaminocarbonyl-$C_1$-$C_6$-alkylgruppe, eine N($C_1$-$C_4$-Alkoxy)-N-($C_1$-$C_4$-alkyl)-aminocarbonyl-$C_1$-$C_6$-alkylgruppe, eine $C_1$-$C_4$-Alkylthiocarbonyl-$C_1$-$C_6$-alkylgruppe, eine $C_1$-$C_4$-Alkoxycarbonylgruppe, eine $C_1$-$C_4$-Alkylcarbony-

loxygruppe, eine $C_1$-$C_4$-Alkoxysulfonyl-$C_1$-$C_6$-alkylgruppe, eine Aminosulfonyl-$C_1$-$C_6$-alkylgruppe, eine $C_1$-$C_4$-Alkylaminosulfonyl-$C_1$-$C_6$-alkylgruppe, eine Di($C_1$-$C_4$)-alkylaminosulfonyl-$C_1$-$C_6$-alkylgruppe

oder

b) eine Hydroxycarbonyl-$C_6$-$C_6$-alkylgruppe, eine Hydroxycarbonyl-$C_1$-$C_6$-alkoxygruppe oder eine Hydroxysulfonyl-$C_1$-$C_6$-alkylgruppe

oder

c) die Phenylgruppe, die Naphthylgruppe, eine 5- oder 6-gliedrige Heteroarylgruppe, eine Phenyl-$C_1$-$C_4$-alkylgruppe, eine Naphthyl-$C_1$-$C_4$-alkylgruppe, eine 5- oder 6-gliedrige Heteroaryl-$C_1$-$C_4$-alkylgruppe, die Phenoxygruppe, die Naphthoxygruppe, eine Phenyl-$C_1$-$C_4$-alkoxygruppe, eine Naphthyl-$C_1$-$C_4$-alkoxy-gruppe, die Benzoylgruppe, die Naphthoylgruppe, eine Phenoxycarbonyl-$C_1$-$C_6$-alkylgruppe, eine Naphthoxycarbonyl-$C_1$-$C_6$-alkylgruppe, eine 5- oder 6-gliedrige Heteroaryloxycarbonyl-$C_1$-$C_6$-alkylgruppe, wobei die Aryl- und Heteroarylteile der genannten Substituenten noch bis zu 3 Halogenatome oder $C_1$-$C_4$-Alkylgruppen oder einen der folgenden Reste tragen können: Hydroxycarbonyl, Nitro, Cyano, Amino, $C_1$-$C_4$-Alkylamino, Di-($C_1$-$C_4$)-alkylamino, $C_1$-$C_4$-Alkoxy oder partiell oder vollständig halogeniertes $C_1$-$C_4$-Alkyl;

oder

d) eine Gruppe

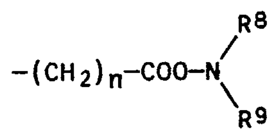 ,

bei der n eine ganze Zahl von 1 bis 6 und $R^8$, $R^9$ $C_1$-$C_3$-Alkyl oder $C_1$-$C_3$-Alkylcarbonyl bedeutet, wobei die Reste $R^8$ und $R^9$ mit dem gemeinsamen Stickstoffatom auch einen 5- oder 6-gliedrigen Ring bilden können,

und/oder ein 1,8-Naphthalindicarbonsäureimid der allgemeinen Formel Ic

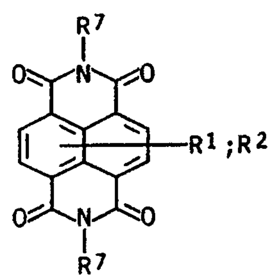

Ic

sowie die pflanzenverträglichen Salze derjenigen Verbindungen Ib und/oder Ic, bei denen $R^1$, $R^2$, $R^3$, $R^4$ Hydroxycarbonyl, Hydroxycarbonyl-$C_1$-$C_4$-alkyl, Hydroxycarbonyl-$C_1$-$C_4$-alkoxy, Hydroxycarbonyl-$C_1$-$C_4$-alkylthio oder Hydroxysulfonyl bedeuten und/oder $R^7$ Hydroxycarbonyl-$C_1$-$C_6$-alkyl, Hydroxycarbonyl-$C_1$-$C_6$-alkoxy oder Hydroxysulfonyl-$C_1$-$C_6$-alkyl bedeutet,

und mindestens einen herbiziden Wirkstoff aus der Gruppe

A) der 2-(4-Heteroaryloxy)- oder 2-(4-Aryloxy)-phenoxyessigsäurederivate der Formel IV,

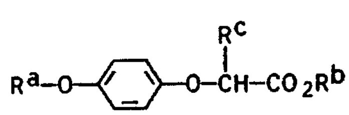

IV

in der die Substituenten folgende Bedeutung haben:

$R^a$ die Phenylgruppe, die Pyridylgruppe, eine Benzoxazylgruppe, eine Benzthiazylgruppe oder eine Benzpyrazinylgruppe, wobei diese aromatischen Ringsysteme bis zu zwei der

folgenden Reste tragen können: Halogen, Nitro, $C_1$-$C_4$-Alkyl, $C_1$-$C_4$-Halogenalkyl und/oder $C_1$-$C_4$-Halogenalkoxy;

$R^b$ Wasserstoff, eine $C_1$-$C_5$-Alkylgruppe, eine $C_3$-$C_5$-Alkylideniminogruppe, eine $C_3$-$C_5$-Alkylideniminooxy-$C_2$-$C_3$-alkylgruppe oder das Äquivalent eines pflanzenverträglichen Kations und

$R^c$ Wasserstoff oder eine Methylgruppe

oder

B) der Cyclohexenonderivate der Formel V,

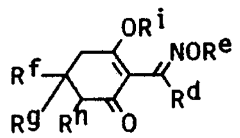

V

in welcher die Substituenten folgende Bedeutung haben:

$R^d$ eine $C_1$-$C_4$-Alkylgruppe;

$R^e$ eine $C_1$-$C_4$-Alkylgruppe, eine $C_3$-$C_4$-Alkenylgruppe, eine $C_3$-$C_4$-Alkinylgruppe oder eine $C_3$-$C_4$-Halogenalkenylgruppe;

einen -($C_1$-$C_4$-Alkyl)-phenyl oder ($C_1$-$C_4$-Alkenyl)-phenylrest, wobei der Alkyl- und Alkenylteil noch bis zu 3 $C_1$-$C_3$-Alkylgruppen und/oder Halogenatome und der Phenylteil bis zu 5 Halogenatome oder einen Benzyloxycarbonyl- oder Phenylrest und/oder bis zu 3 der folgenden Substituenten tragen kann: Nitro, Cyano, $C_1$-$C_4$-Alkyl, $C_1$-$C_4$-Alkoxy, $C_1$-$C_4$-Alkylthio, partiell oder vollständig halogeniertes $C_1$-$C_4$-Alkyl, partiell oder vollständig halogeniertes $C_1$-$C_4$-Alkoxy, Carboxyl oder $C_1$-$C_4$-Alkoxycarbonyl; die Thienylgruppe, die noch ein Halogenatom tragen kann;

$R^f$ eine $C_1$-$C_4$-Alkylgruppe, welche einfach durch $C_1$-$C_4$-Alkylthio oder $C_1$-$C_4$-Alkoxy substituiert sein kann;

ein 5- oder 6-gliedriges gesättigtes oder einfach ungesättigtes Ringsystem, welches neben Kohlenstoffgliedern ein Sauerstoff-, ein Schwefelatom oder eine Sulfoxid- oder Sulfongruppe enthalten kann, wobei dieser Ring bis zu drei der folgenden Reste tragen kann: Hydroxy, Halogen, $C_1$-$C_4$-Alkyl, $C_1$-$C_4$-Halogenalkyl, $C_1$-$C_4$-Alkoxy und/oder $C_1$-$C_4$-Alkylthio;

einen 10-gliedrigen gesättigten oder einfach ungesättigten Heterocyclus, welcher zwei Sauerstoffatome oder Schwefelatome enthält und durch bis zu drei $C_1$-$C_4$-Alkylgruppen und/oder Methoxygruppen substituiert sein kann;

die Phenylgruppe oder die Pyridylgruppe, wobei diese Gruppen bis zu drei der folgenden Reste tragen können: $C_1$-$C_4$-Alkyl, $C_1$-$C_4$-Halogenalkyl, $C_1$-$C_4$-Alkoxy, $C_1$-$C_4$-Alkylthio, $C_3$-$C_6$-Alkenyloxy, $C_3$-$C_6$-Alkinyloxy, $C_1$-$C_4$-Alkoxy-$C_1$-$C_3$-alkyl, $C_1$-$C_4$-Dialkoxy-$C_1$-$C_3$-alkyl, Formyl, Halogen und/oder Benzoylamino;

die Pyrrolylgruppe, die Pyrazolyl-, Thiazolyl- oder Isoxazolylgruppe, die jeweils noch eine $C_1$-$C_4$-Alkylgruppe tragen kann;

$R^g$ Wasserstoff, Hydroxy oder, wenn $R^f$ die Bedeutung $C_1$-$C_6$-Alkylgruppe hat, eine $C_1$-$C_6$-Alkylgruppe;

$R^h$ Wasserstoff, die Cyanogruppe, ein Halogenatom, eine $C_1$-$C_4$-Alkoxycarbonylgruppe oder eine Gruppe

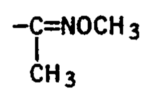

und

$R^i$ Wasserstoff oder das Äquivalent eines umweltverträglichen Kations.

**4.** Herbizide Mittel nach Anspruch 3, enthaltend ein substituiertes 1,8-Naphthalindicarbonsäureimid Ia und/oder Ib und/oder Ic gemäß Anspruch 3 und ein Herbizid IV oder ein Herbizid V im Gewichtsverhältnis 0,01:1 bis 10:1.

5. Verfahren zur selektiven Bekämpfung von unerwünschtem Pflanzenwuchs, dadurch gekennzeichnet, daß man ein substituiertes 1,8-Naphthalindicarbonsäureimid Ia und/oder Ib und/oder Ic und
   A) ein 2-(4-Heteroaryloxy)- oder 2-(4-Aryloxy)-phenoxyessigsäurederivat der Formel IV oder
   B) ein Cyclohexenonderivat der Formel V gemäß Anspruch 3 vor, bei oder nach der Aussaat der Kulturpflanzen, vor oder während des Auflaufens der Kulturpflanzen gleichzeitig oder nacheinander ausbringt.

6. Verfahren zur selektiven Bekämpfung von unerwünschtem Pflanzenwuchs, dadurch gekennzeichnet, daß man die Blätter der Kulturpflanzen und der unerwünschten Pflanzen im Nachauflaufverfahren mit einem substituiertem 1,8-Naphthalindicarbonsäureimid Ia und/oder Ib und/oder Ic und
   A) mit einem 2-(4-Heteroaryloxy)- oder 2-(4-Aryloxy)-phenoxyessigsäurederivat der Formel IV oder
   B) mit einem Cyclohexenonderivat der Formel V gemäß Anspruch 3 gleichzeitig oder nacheinander behandelt.

7. Verfahren zur Verhinderung von Schädigungen von Kulturpflanzen durch herbizide 2-(4-Heteroaryloxy)- oder 2-(4-Aryloxy)-phenoxyessigsäurederivate der Formel IV oder durch herbizide Cyclohexenonderivate der Formel V gemäß Anspruch 3, dadurch gekennzeichnet, daß man das Saatgut der Kulturpflanzen mit einer antagonistisch wirksamen Menge eines substituierten 1,8-Naphthalindicarbonsäureimids der Formel Ia und/oder Ib und/oder Ic gemäß Anspruch 3 behandelt.

8. Verfahren gemäß den Anspruchen 5 bis 7, dadurch gekennzeichnet, daß die Kulturpflanzen Gerste, Weizen, Mais, Kultursorghum und Reis sind.

Patentansprüche für folgenden Vertragsstaat: ES

1. Herbizide Mittel, enthaltend mindestens ein substituiertes 1,8-Naphthalindicarbonsäureimid der allgemeinen Formel Ia

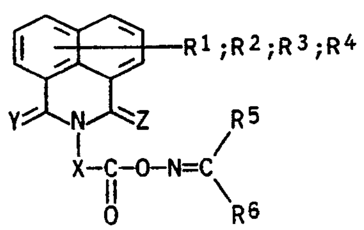

Ia

in der die Substituenten die folgende Bedeutung haben:

$R^1$ - $R^4$ Wasserstoff, Halogen, Hydroxy, Mercapto, Cyano, Thiocyanato, Nitro, $C_1$-$C_6$-Alkylgruppen, die unsubstituiert oder partiell oder vollständig halogeniert sein können, $C_2$-$C_6$-Alkenylgruppen, $C_2$-$C_6$-Alkinylgruppen, $C_3$-$C_8$-Cycloalkyl-oder $C_3$-$C_8$-Cycloalkoxygruppen, die Aminogruppe, $C_1$-$C_8$-Alkylamino- oder Di-($C_1$-$C_6$)-alkylaminogruppen, Amino-$C_1$-$C_4$-alkylgruppen, $C_1$-$C_6$-Alkylamino-$C_1$-$C_4$-alkyl-oder Di-($C_1$-$C_6$)-alkylamino-$C_1$-$C_4$-alkylgruppen, $C_1$-$C_4$-Alkoxygruppen, $C_1$-$C_4$-Alkylthiogruppen, $C_1$-$C_4$-Alkoxy-$C_1$-$C_4$-alkylgruppen, $C_1$-$C_4$-Alkylthio-$C_1$-$C_4$-alkylgruppen, $C_1$-$C_6$-Alkylcarbonyl- oder $C_1$-$C_6$-Alkylcarbonyloxygruppen, $C_1$-$C_6$-Alkoxycarbonylgruppen, die Hydroxycarbonylgruppe, Hydroxycarbonyl-$C_1$-$C_4$-alkylgruppen, Hydroxycarbonyl-$C_1$-$C_4$-alkoxy- oder Hydroxycarbonyl-$C_1$-$C_4$-alkylthiogruppen, die Hydroxysulfonylgruppe, $C_1$-$C_6$-Alkylsulfinyl- oder $C_1$-$C_6$-Alkylsulfonylgruppen, $C_1$-$C_4$-Alkoxysulfonylgruppen, die Aminosulfonylgruppe, $C_1$-$C_4$-Alkylaminosulfonyl- oder Di-($C_1$-$C_4$)-alkylaminosulfonylgruppen, die Hydrazinogruppe, die bis zu drei $C_1$-$C_4$-Alkylreste tragen kann, die Phenylgruppe, Phenyl-$C_1$-$C_3$-alkylgruppen, die Naphthylgruppe, Naphthyl-$C_1$-$C_3$-alkylgruppen, 5- oder 6-gliedrige Heteroaryl- oder Heteroaryl-$C_1$-$C_3$-alkylgruppen, die Phenoxy- oder Naphthoxygruppe, die Phenylthio- oder Naphthylthiogruppe, die Phenoxycarbonyl-oder Naphthoxycarbonylgruppe, die Phenylcarbonyl-oder Naphthylcarbonylgruppe, die Phenylcarbonyloxy-oder Naphthylcarbonyloxygruppe, Phenyl-$C_1$-$C_3$-alkoxy-oder Naphthyl-$C_1$-$C_3$-alkoxygruppen, 5- oder 6-gliedrige Heteroaryl-$C_1$-$C_3$-alkoxygruppen, die Phenylsulfinyl- oder Naphthylsulfinylgruppe, 5- oder 6-gliedrige Heteroarylsulfinylgruppen, die Phenylsulfonyl-oder

Naphthylsulfonylgruppe, 5- oder 6-gliedrige Heteroarylsulfonylgruppen, die Phenoxysulfonyl- oder Naphthoxysulfonylgruppe, 5- oder 6-gliedrige Heteroaryloxysulfonylgruppen, wobei die Aryl- und Heteroarylteile der genannten Substituenten noch bis zu drei der folgenden Substituenten tragen können: Halogen, Cyano, Nitro, $C_1$-$C_4$-Alkyl, $C_1$-$C_4$-Alkoxy, Amino, $C_1$-$C_6$-Alkylamino, Di-($C_1$-$C_6$)-alkylamino oder partiell oder vollständig halogeniertes $C_1$-$C_4$-Alkyl;

x eine $C_1$-$C_6$-Alkylenkette, die noch einen der folgenden Reste tragen kann: Halogen, Hydroxy, Mercapto, $C_1$-$C_4$-Alkyl, $C_1$-$C_4$-Alkoxy, $C_1$-$C_4$-Alkylthio, Di-($C_1$-$C_4$)-alkylamino, $C_1$-$C_4$-Alkoxycarbonyl, Cyano, $C_1$-$C_6$-Alkylcarbonyl, $C_1$-$C_6$-Alkylcarbonyloxy, Phenyl, Naphthyl, 5- oder 6-gliedriges Heteroaryl, Phenyl-($C_1$-$C_3$)-alkyl, Naphthyl-($C_1$-$C_3$)-alkyl oder 5- oder 6-gliedriges Heteroaryl-($C_1$-$C_3$)-alkyl, wobei die Aryl- und Heteroarylteile der letztgenannten sechs Reste noch bis zu 3 Halogenatome oder $C_1$-$C_4$-Alkylgruppen tragen können;

$R^5$ Wasserstoff, Cyano, eine $C_1$-$C_6$-Alkylgruppe, eine $C_2$-$C_6$-Alkenylgruppe, eine $C_1$-$C_4$-Alkoxy-$C_1$-$C_4$-alkylgruppe, eine $C_1$-$C_4$-Alkylthio-$C_1$-$C_4$-alkylgruppe, eine $C_1$-$C_4$-Alkoxycarbonylgruppe, die Acetylgruppe, eine $C_3$-$C_6$-Cycloalkylgruppe, die Benzoyl-, Naphthoyl- oder eine 5- oder 6-gliedrige Heteroaroylgruppe, die Phenyl- oder Naphthylgruppe, eine Phenyl-$C_1$-$C_3$-alkyl- oder Naphthyl-$C_1$-$C_3$-alkylgruppe, eine 5- oder 6-gliedrige Heteroaryl- oder Heteroaryl-$C_1$-$C_3$-alkylgruppe, wobei die Aryl- und Heteroarylteile der letztgenannten sechs Gruppen noch bis zu drei der folgenden Reste tragen können: Halogen, Hydroxy, Cyano, Trifluormethyl, $C_1$-$C_6$-Alkoxy, das unsubstituiert oder partiell oder vollständig halogeniert sein kann, $C_1$-$C_6$-Alkylthio, $C_1$-$C_6$-Alkyl, Di-($C_1$-$C_3$)-alkylamino oder Acetamino;

$R^6$ Wasserstoff, Cyano, eine $C_1$-$C_6$-Alkylgruppe, die unsubstituiert oder partiell oder vollständig halogeniert sein kann, eine $C_1$-$C_4$-Alkoxygruppe, eine $C_1$-$C_4$-Alkoxy-$C_1$-$C_4$-alkylgruppe, eine Diazolylmethyl- oder Triazolylmethylgruppe, eine $C_1$-$C_6$-Alkoxycarbonylgruppe, die Furyl- oder Tetrahydrofurylgruppe, die Thienylgruppe, eine Dihydropyranyl- oder Dihydrothiopyranylgruppe, die Tetrahydropyranyl-oder Tetrahydrothiopyranylgruppe, oder

eine Phenylgruppe, wenn $R^5$ Wasserstoff, Methyl oder Acetyl bedeutet;

oder

zusammen mit dem Rest $R^5$ und dem gemeinsamen C-Atom einen $C_3$-$C_{12}$-Cycloalkylrest, einen 4-Oxocyclohexadienylrest oder einen 5- oder 6-gliedrigen Ring, der gesättigt oder partiell oder vollständig ungesättigt sein kann und ein Sauerstoff- oder Schwefelatom als Heteroatom enthalten kann und/oder der noch bis zu drei $C_1$-$C_3$-Alkyl- oder $C_1$-$C_3$-Alkoxygruppen oder einen anellierten Benzolring tragen kann;

Y, Z Sauerstoff oder Schwefel;

mit der Maßgabe, daß $R^5$ und $R^6$ nicht gleichzeitig Wasserstoff bedeuten, sowie die pflanzenverträglichen Salze derjenigen Verbindungen Ia, bei denen $R^1$ ; $R^2$; $R^3$; $R^4$ Hydroxycarbonyl, Hydroxycarbonyl-$C_1$-$C_4$-alkyl, Hydroxycarbonyl-$C_1$-$C_4$-alkoxy, Hydroxycarbonyl-$C_1$-$C_4$-alkylthio oder Hydroxysulfonyl bedeuten, und/oder ein 1,8-Naphthalindicarbonsäureimid der allgemeinen Formel Ib

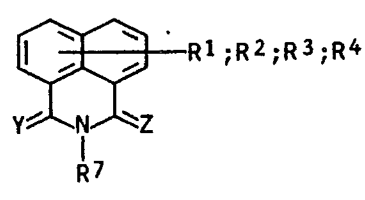

Ib

in der $R^7$ die folgende Bedeutung hat:

a) Wasserstoff, Hydroxy, Cyano, eine $C_1$-$C_6$-Alkylgruppe, die unsubstituiert oder partiell oder vollständig halogeniert sein kann, eine $C_1$-$C_6$-Hydroxyalkylgruppe, eine $C_2$-$C_6$-Alkenylgruppe, eine $C_1$-$C_4$-Alkoxygruppe, eine $C_1$-$C_4$-Alkoxy-$C_1$-$C_4$-alkylgruppe, eine $C_1$-$C_4$-Alkylthio-$C_1$-$C_4$-alkylgruppe, die Aminogruppe, eine $C_1$-$C_4$-Alkylaminogruppe, eine Di-($C_1$-$C_4$)-alkylaminogruppe, eine $C_3$-$C_8$-Cycloalkylgruppe, eine $C_3$-$C_8$-Cycloalkoxygruppe, eine Acylgruppe, eine $C_1$-$C_4$-Alkoxycarbonyl-$C_1$-$C_6$-alkylgruppe, eine Aminocarbonyl-$C_1$-$C_6$-alkylgruppe, eine $C_1$-$C_4$-Alkylaminocarbonyl-$C_1$-$C_6$-alkylgruppe, eine Di-($C_1$-$C_4$)-alkylaminocarbonyl-$C_1$-$C_6$-alkylgruppe, eine $C_1$-$C_4$-Alkoxyaminocarbonyl-$C_1$-

$C_6$-alkylgruppe, eine N-($C_1$-$C_4$-Alkoxy)-N-($C_1$-$C_4$-alkyl)-aminocarbonyl-$C_1$-$C_6$-alkylgruppe, eine $C_1$-$C_4$-Alkylthiocarbonyl-$C_1$-$C_6$-alkylgruppe, eine $C_1$-$C_4$-Alkoxycarbonylgruppe, eine $C_1$-$C_4$-Alkylcarbonyloxygruppe, eine $C_1$-$C_4$-Alkoxysulfonyl-$C_1$-$C_6$-alkylgruppe, eine Aminosulfonyl-$C_1$-$C_6$-alkylgruppe, eine $C_1$-$C_4$-Alkylaminosulfonyl-$C_1$-$C_6$-alkylgruppe, eine Di-($C_1$-$C_4$)-alkylaminosulfonyl-$C_1$-$C_6$-alkylgruppe

oder

b) eine Hydroxycarbonyl-$C_1$-$C_6$-alkylgruppe, eine Hydroxycarbonyl-$C_1$-$C_6$-alkoxygruppe oder eine Hydroxysulfonyl-$C_1$-$C_6$-alkylgruppe

oder

c) die Phenylgruppe, die Naphthylgruppe, eine 5- oder 6-gliedrige Heteroarylgruppe, eine Phenyl-$C_1$-$C_4$-alkylgruppe, eine Naphthyl-$C_1$-$C_4$-alkylgruppe, eine 5- oder 6-gliedrige Heteroaryl-$C_1$-$C_4$-alkylgruppe, die Phenoxygruppe, die Naphthoxygruppe, eine Phenyl-$C_1$-$C_4$-alkoxygruppe, eine Naphthyl-$C_1$-$C_4$-alkoxygruppe, die Benzoylgruppe, die Naphthoylgruppe, eine Phenoxycarbonyl-$C_1$-$C_6$-alkylgruppe, eine Naphthoxycarbonyl-$C_1$-$C_6$-alkylgruppe, eine 5- oder 6-gliedrige Heteroaryloxycarbonyl-$C_1$-$C_6$-alkylgruppe, wobei die Aryl- und Heteroarylteile der genannten Substituenten noch bis zu 3 Halogenatome oder $C_1$-$C_4$-Alkylgruppen oder einen der folgenden Reste tragen können: Hydroxycarbonyl, Nitro, Cyano, Amino, $C_1$-$C_4$-Alkylamino, Di-($C_1$-$C_4$)-alkylamino, $C_1$-$C_4$-Alkoxy oder partiell oder vollständig halogeniertes $C_1$-$C_4$-Alkyl;

oder

d) eine Gruppe

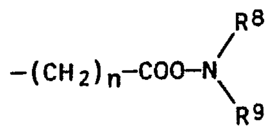 ,

bei der n eine ganze Zahl von 1 bis 6 und $R^8$, $R^9$ $C_1$-$C_3$-Alkyl oder $C_1$-$C_3$-Alkylcarbonyl bedeutet, wobei die Reste $R^8$ und $R^9$ mit dem gemeinsamen Stickstoffatom auch einen 5- oder 6-gliedrigen Ring bilden können,

und/oder ein 1,8-Naphthalindicarbonsäureimid der allgemeinen Formel Ic

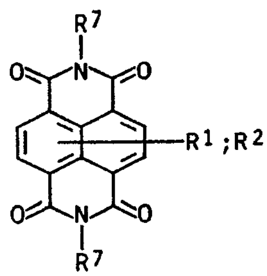

Ic

sowie die pflanzenverträglichen Salze derjenigen Verbindungen Ib und/oder Ic, bei denen $R^1$, $R^2$, $R^3$, $R^4$ Hydroxycarbonyl, Hydroxycarbonyl-$C_1$-$C_4$-alkyl, Hydroxycarbonyl-$C_1$-$C_4$-alkoxy, Hydroxycarbonyl-$C_1$-$C_4$-alkylthio oder Hydroxysulfonyl bedeuten und/oder $R^7$ Hydroxycarbonyl-$C_1$-$C_6$-alkyl, Hydroxycarbonyl-$C_1$-$C_6$-alkoxy oder Hydroxysulfonyl-$C_1$-$C_6$-alkyl bedeutet,

und mindestens einen herbiziden Wirkstoff aus der Gruppe

A) der 2-(4-Heteroaryloxy)- oder 2-(4-Aryloxy)-phenoxyessigsäurederivate der Formel IV,

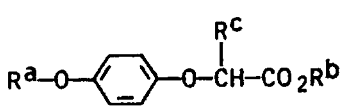

IV

in der die Substituenten folgende Bedeutung haben:

$R^a$ die Phenylgruppe, die Pyridylgruppe, eine Benzoxazylgruppe, eine Benzthiazylgruppe oder eine Benzpyrazinylgruppe, wobei diese aromatischen Ringsysteme bis zu zwei der

folgenden Reste tragen können: Halogen, Nitro, $C_1$-$C_4$-Alkyl, $C_1$-$C_4$-Halogenalkyl und/oder $C_1$-$C_4$-Halogenalkoxy;

$R^b$ Wasserstoff, eine $C_1$-$C_5$-Alkylgruppe, eine $C_3$-$C_5$-Alkylideniminogruppe, eine $C_3$-$C_5$-Alkylideniminooxy-$C_2$-$C_3$-alkylgruppe oder das Äquivalent eines pflanzenverträglichen Kations und

$R^c$ Wasserstoff oder eine Methylgruppe

oder

B) der Cyclohexenonderivate der Formel V,

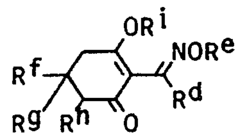

V

in welcher die Substituenten folgende Bedeutung haben:

$R^d$ eine $C_1$-$C_4$-Alkylgruppe;

$R^e$ eine $C_1$-$C_4$-Alkylgruppe, eine $C_3$-$C_4$-Alkenylgruppe, eine $C_3$-$C_4$-Alkinylgruppe oder eine $C_3$-$C_4$-Halogenalkenylgruppe;
einen -($C_1$-$C_4$-Alkyl)-phenyl oder -($C_1$-$C_4$-Alkenyl)-phenylrest, wobei der Alkyl- und Alkenylteil noch bis zu 3 $C_1$-$C_3$-Alkylgruppen und/oder Halogenatome und der Phenylteil bis zu 5 Halogenatome oder einen Benzyloxycarbonyl- oder Phenylrest und/oder bis zu 3 der folgenden Substituenten tragen kann: Nitro, Cyano, $C_1$-$C_4$-Alkyl, $C_1$-$C_4$-Alkoxy, $C_1$-$C_4$-Alkylthio, partiell oder vollständig halogeniertes $C_1$-$C_4$-Alkyl, partiell oder vollständig halogeniertes $C_1$-$C_4$-Alkoxy, Carboxyl oder $C_1$-$C_4$-Alkoxycarbonyl;
die Thienylgruppe, die noch ein Halogenatom tragen kann;

$R^f$ eine $C_1$-$C_4$-Alkylgruppe, welche einfach durch $C_1$-$C_4$-Alkylthio oder $C_1$-$C_4$-Alkoxy substituiert sein kann;
ein 5- oder 6-gliedriges gesättigtes oder einfach ungesättigtes Ringsystem, welches neben Kohlenstoffgliedern ein Sauerstoff-, ein Schwefelatom oder eine Sulfoxid- oder Sulfongruppe enthalten kann, wobei dieser Ring bis zu drei der folgenden Reste tragen kann: Hydroxy, Halogen, $C_1$-$C_4$-Alkyl, $C_1$-$C_4$-Halogenalkyl, $C_1$-$C_4$-Alkoxy und/oder $C_1$-$C_4$-Alkylthio;
einen 10-gliedrigen gesättigten oder einfach ungesättigten Heterocyclus, welcher zwei Sauerstoffatome oder Schwefelatome enthält und durch bis zu drei $C_1$-$C_4$-Alkylgruppen und/oder Methoxygruppen substituiert sein kann;
die Phenylgruppe oder die Pyridylgruppe, wobei diese Gruppen bis zu drei der folgenden Reste tragen können: $C_1$-$C_4$-Alkyl, $C_1$-$C_4$-Halogenalkyl, $C_1$-$C_4$-Alkoxy, $C_1$-$C_4$-Alkylthio, $C_3$-$C_6$-Alkenyloxy, $C_3$-$C_6$-Alkinyloxy, $C_1$-$C_4$-Alkoxy-$C_1$-$C_3$-alkyl, $C_1$-$C_4$-Dialkoxy-$C_1$-$C_3$-alkyl, Formyl, Halogen und/oder Benzoylamino;
die Pyrrolylgruppe, die Pyrazolyl-, Thiazolyl- oder Isoxazolylgruppe, die jeweils noch eine $C_1$-$C_4$-Alkylgruppe tragen kann;

$R^g$ Wasserstoff, Hydroxy oder, wenn $R^f$ die Bedeutung $C_1$-$C_6$-Alkylgruppe hat, eine $C_1$-$C_6$-Alkylgruppe;

$R^h$ Wasserstoff, die Cyanogruppe, ein Halogenatom, eine $C_1$-$C_4$-Alkoxycarbonylgruppe oder eine Gruppe

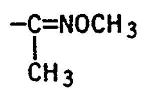

und

$R^i$ Wasserstoff oder das Äquivalent eines umweltverträglichen Kations.

**2.** Verfahren zur Herstellung der Verbindungen Ia gemäß Anspruch 1, dadurch gekennzeichnet, daß man ein Säurehalogenid der allgemeinen Formel II

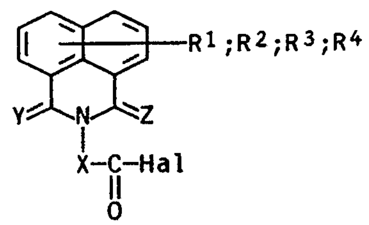

II,

wobei Hal für Fluor, Chlor oder Brom steht, mit einem Oxim der allgemeinen Formel III

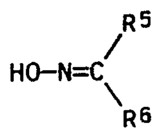

III

umsetzt.

3. Herbizide Mittel nach Anspruch 1, enthaltend ein substituiertes 1,8-Naphthalindicarbonsäureimid Ia und/oder Ib und/oder Ic und ein Herbizid IV oder ein Herbizid V im Gewichtsverhältnis 0,01:1 bis 10:1.

4. Verfahren zur selektiven Bekämpfung von unerwünschtem Pflanzenwuchs, dadurch gekennzeichnet, daß man ein substituiertes 1,8-Naphthalindicarbonsäureimid Ia und/oder Ib und/oder Ic und
A) ein 2-(4-Heteroaryloxy)- oder 2-(4-Aryloxy)-phenoxyessigsäurederivat der Formel IV oder
B) ein Cyclohexenonderivat der Formel V gemäß Anspruch 1 vor, bei oder nach der Aussaat der Kulturpflanzen, vor oder während des Auflaufens der Kulturpflanzen gleichzeitig oder nacheinander ausbringt.

5. Verfahren zur selektiven Bekämpfung von unerwünschtem Pflanzenwuchs, dadurch gekennzeichnet, daß man die Blätter der Kulturpflanzen und der unerwünschten Pflanzen im Nachauflaufverfahren mit einem substituiertem 1,8-Naphthalindicarbonsäureimid Ia und/oder Ib und/oder Ic und
A) mit einem 2-(4-Heteroaryloxy)- oder 2-(4-Aryloxy)-phenoxyessigsäurederivat der Formel IV oder
B) mit einem Cyclohexenonderivat der Formel V gemäß Anspruch 1 gleichzeitig oder nacheinander behandelt.

6. Verfahren zur Verhinderung von Schädigungen von Kulturpflanzen durch herbizide 2-(4-Heteroaryloxy)- oder 2-(4-Aryloxy)-phenoxyessigsäurederivate der Formel IV oder durch herbizide Cyclohexenonderivate der Formel V gemäß Anspruch 1, dadurch gekennzeichnet, daß man das Saatgut der Kulturpflanzen mit einer antagonistisch wirksamen Menge eines substituierten 1,8-Naphthalindicarbonsäureimids der Formel Ia und/oder Ib und/oder Ic gemäß Anspruch 1 behandelt.

7. Verfahren gemäß den Ansprüchen 4 bis 6, dadurch gekennzeichnet, daß die Kulturpflanzen Gerste, Weizen, Mais, Kultursorghum und Reis sind.